# EUROPEAN PATENT APPLICATION

(11) **EP 4 818 208 A1**
(43) Date of publication of application: **07.10.2026**
(21) Application number: 25382321.5
(22) Date of filing: 31.03.2025
(51) Int. Cl.: C07K 16/28, A61P 35/00, A61K 40/31

(54) **CLAUDIN 18.2 TARGETING MOIETY FOR THE TREATMENT OF CLAUDIN-POSITIVE CANCERS**

(71) Applicant: Universidade de Vigo, 36310 Vigo, Pontevedra (ES); Galaria, Empresa Pública de Servizos Sanitarios S.A., 15703 Santiago de Compostela A Coruña (ES); Hospital Clínic de Barcelona, 08036 Barcelona (ES); Fundació de Recerca Clínic Barcelona-Institut d'Investigacions Biomèdiques August Pi I Sunyer, 08036 Barcelona (ES)
(72) Inventor: GONZÁLEZ FERNÁNDEZ, María África, 36310 Vigo (ES); VERÍSIMO GARCÍA, Joel, 15703 Santiago de Compostela (ES); JUAN OTERO, Manuel, 08036 Barcelona (ES); BALIU PIQUÉ, Mariona, 15703 Santiago de Compostela (ES)
(74) Representative: Hoffmann Eitle

(57) **Abstract**

The present invention refers to a Claudin 18.2 targeting moiety specifically binds to the amino acid sequence of SEQ ID NO: 1.

## Description

### Technical Field

The present invention provides therapeutics for the treatment of Claudin 18.2-positive cancer. In particular, the present invention provides a Claudin 18.2 targeting moiety.

### Background art

T-cells are major effectors in cancer immunotherapy. The effector functions of T-cells can be substantially boosted by redirecting them against tumor antigens *via* genetic engineering of T-cell receptors or chimeric antigen receptors (CARs) to generate tumor-reactive T-cells for adoptive transfer. CARs are fusion molecules typically containing an extracellular single chain variable fragment (scFv) of a monoclonal antibody specific to a surface molecule on the tumor cell, a spacer domain that provides flexibility and optimizes effector-target cell engagement, a transmembrane domain, and the T-cell signaling domains 4-1BB (or CD28) and CD3ζ, which drive survival/proliferation and cytotoxicity, respectively. CAR T-cell (CART) therapy has revolutionized cancer treatment, especially for relapse/refractory (R/R) B-cell acute lymphoblastic leukemia (B-ALL) and B-cell lymphomas, for which a specific and relatively safe target antigen exists (CD19).

However, CAR-T therapies for solid tumors are still in their infancy, and our current understanding of the mechanisms underlying successful adoptive immunotherapy and the clinic-biological parameters predicting response to immunotherapy remain elusive. Of note, CAR-T therapies for non-B-cell malignancies are lagging owing to the absence of safe and specific targets, and/or CAR-T delivery-associated challenges.

Claudins are integral membrane proteins located within the tight junctions of epithelia and endothelia. The claudin (CLDN) family of transmembrane proteins plays a critical role in the maintenance of epithelial and endothelial tight junctions and might also play a role in the maintenance of the cytoskeleton and in cell signaling.

The Claudin 18 molecule is an integral transmembrane protein (tetraspanin) having four membrane spanning hydrophobic regions and two extracellular loops (loop 1 embraced by hydrophobic region 1 and hydrophobic region 2; **loop 2** embraced by hydrophobic regions 3 and 4). Claudin 18 exists in two different splice variants (18.1 and 18.2) and differ in the N-terminal portion. Claudin 18.1 is selectively expressed on cells of normal lung, whereas Claudin 18.2 is usually expressed on gastric cells. However, Claudin 18.2 expression in normal stomach is restricted to the differentiated short-lived cells of stomach epithelium. Claudin 18.2 over-expression has been identified in various tumor tissues, especially in gastric cancer and pancreatic adenocarcinoma, esophageal carcinoma, bronchial carcinoma, ovary and metastasis associated to gastric cancer (peritoneal and/or lymphatic).

Claudin 18.2 is thus a valuable target for diagnosis and treatment of primary tumors. The differential expression of Claudin 18.2 between cancer and normal cells, together with its surface membrane localization, makes Claudin 18.2 an attractive target for cancer immunotherapy.

The present invention aims to provide a novel therapy for treating Claudin 18.2-positive cancers.

### Figure Legends

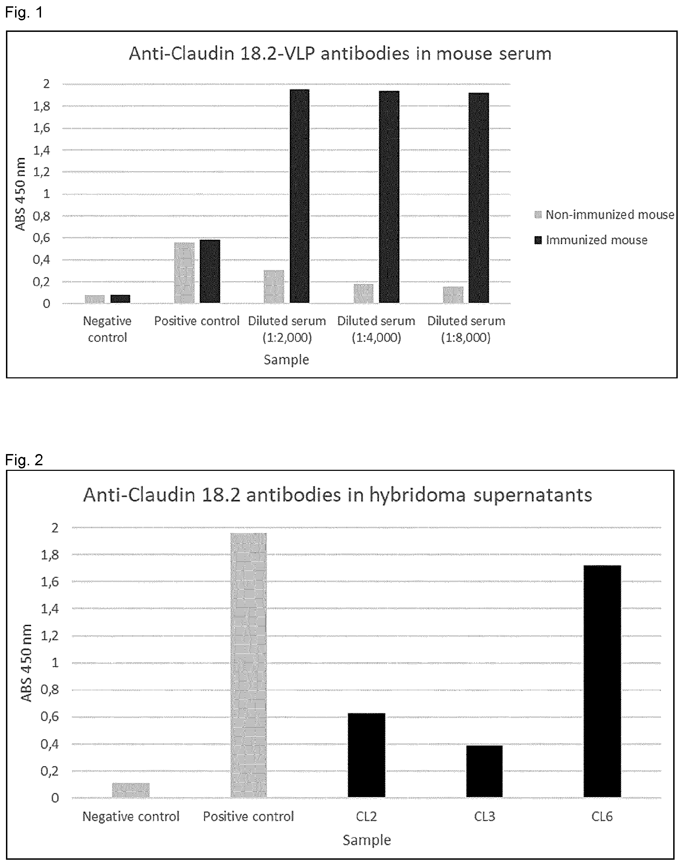

**Figure 1****:** Detection of specific antibodies against Claudin 18.2-VLP in the serum of non-immunized (grey) and immunized (black) mice at dilutions of 1:2,000, 1:4,000 and 1:8,000 in PBS-bovine serum albumin (BSA) (1%). Absorbance values from indirect ELISA are shown. PBS-BSA (1%) served as negative control, while a commercial monoclonal anti-Claudin 18.2 antibody was used as positive control.
**Figure 2****:** Antibodies produced by murine hybridomas that recognize Claudin 18.2 protein. Absorbance values from indirect ELISA are shown. RPMI served as negative control, and serum from immunized mouse diluted 1:10,000 in PBS-BSA (1%) as positive control.

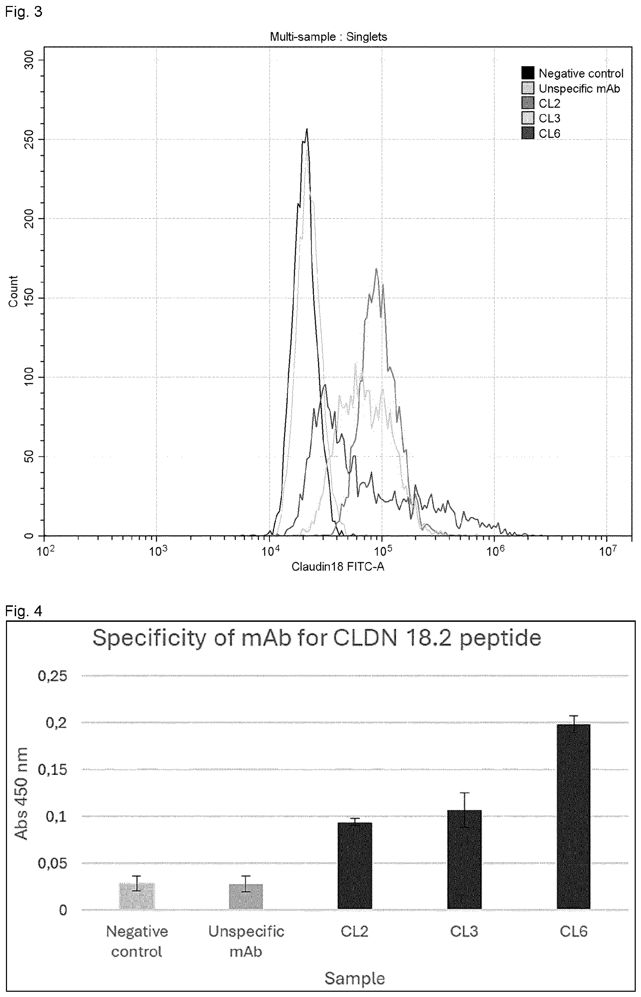

**Figure 3****:** Flow cytometry analysis of Claudin 18.2 transfected MIAPaCa-2 tumoral cells using monoclonal antibodies produced by hybridomas CL2 (red), CL3 (green) and CL6(blue). Cells unstained (black) or incubated with an unspecific IgG (grey) were used as negative controls.. Events relative to fluorescence intensity from FITC fluorochrome labelling are shown.
**Figure 4****:** Selected CL2, CL3 and CL6 monoclonal antibodies recognize the peptide derived from human Claudin 18.2 protein. Absorbance values from indirect ELISA are shown. PBS and an unspecific IgG served as negative controls (grey).

### Definitions

The term "Claudin 18" relates to Claudin 18 protein, preferably human Claudin 18 protein, and includes any splice variants such as Claudin 18.1 and Claudin 18.2. Claudin 18.1 and Claudin 18.2 differ in the N-terminal portion which comprises the first transmembrane (TM) region and loop, whereas the primary protein sequence of the C-terminus is identical. The term "Claudin 18.2" preferably relates to human Claudin 18.2, and, in particular, to a protein comprising the amino acid sequence according to SEQ ID NO: 1 of the sequence listing or a variant of said amino acid sequence. The term "variant" according to the invention refers, in particular, to mutants, splice variants, conformations, isoforms, allelic variants, species variants and species homologs, in particular those which are naturally present. The term "Claudin 18.2" also encompasses any post-translationally modified variants and conformation variants.

The term "Claudin 18.2 targeting moiety" refers to a substance or molecule or part thereof that is able to bind, preferably specifically bind, to Claudin 18.2, preferably to SEQ ID NO: 1

The term "affibody" refers to a protein that is derived from the Z domain of protein A and that been engineered to bind to a specific target.

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies. A typical type of antibody comprises at least two heavy chains ("HC") and two light chains ("LC") interconnected by disulfide bonds.

Each "heavy chain" comprises a "heavy chain variable domain" (abbreviated herein as "VH") and a "heavy chain constant domain" (abbreviated herein as "CH"). The heavy chain constant domain typically comprises three constant domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW").

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

The precise amino acid sequence boundaries of a given CDR can be determined using any of a number of well-known schemes, preferably using the IMGT numbering system described by Lefranc, M.-P., "The IMGT unique numbering for immunoglobulins, T cell Receptors and Ig-like domains", The Immunologist, 7, 132-136 (1999). However, it is known that the exact sequence and boundaries of a given CDR may vary depending on the numbering system used. Thus, it is considered to be encompassed by the present invention all CDR variations covering not only the CDR sequences described herein, but also at least +3, +2, +1 amino acid placed upstream (i.e., in the N-terminal direction), and downstream (i.e., in the C-terminal direction) of the sequence.

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

An antibody can be of any the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target.

The term "antigen-binding fragment" or "Fab" refers to an antibody fragment comprising one constant and one variable domain of each of the heavy and light chain. A Fab fragment may be obtained by digesting an intact monoclonal antibody with papain.

The term "designed ankyrin repeat proteins" or "DARPin" refers to a protein that is derived from an ankyrin repeat that has been engineered to bind to a specific target.

The term "fynomer" refers to a protein that is derived from the SH3 domain of human Fyn kinase that has been engineered to bind to a specific target. The term "monobody" refers to a protein that is derived from a fibronectin type II domain that has been engineered to bind to a specific target.

The term "nanobody" refers to a protein comprising the soluble single antigen-binding V-domain of a heavy chain antibody, preferably a camelid heavy chain antibody.

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv).

The term "peptide aptamer" refers to a short, 5-20 amino acid residue sequence, that can bind to a specific target.

The term "repebody" refers to a protein that is derived from a leucine-rich repeat module and that been engineered to bind to a specific target.

The term "chimeric antigen receptor" or "CAR" refers to a synthetic receptor that targets alpha-beta T-cells or other effector cells, such as for example natural killer (NK) cells, gamma delta T cells, macrophages or others, to a chosen antigen and reprograms T cell function, metabolism and persistence. Within the context of a CAR anti-Claudin 18.2, the Claudin 18.2-targeting moiety comprised in the CAR targets a Claudin 18.2 -positive cell, preferably a cancer cell. Within the context of a CAR, it is to be understood that the Claudin 18.2-targeting moiety is genetically encodable.

The term "costimulatory signaling domain" refers to a signaling moiety that provides to T cells a signal which, in addition to the primary signal provided by for instance the CD3ζ chain of the TCR/CD3 complex, mediates a T cell response, including, but not limited to, activation, proliferation, differentiation, cytokine secretion, and others. A co-stimulatory domain can include all or a portion of, but is not limited to, CD27, CD28, 4-1BB (CD137), OX40 (CD134), CD30, CD40, 1COS, lymphocyte function-associated antigen-1 (LFA-1), CD2, CD7, LIGHT, NKG2C, B7-H3, and a ligand that specifically binds with CD83. In some embodiments, the costimulatory signaling domain is an intracellular signaling domain that interacts with other intracellular mediators to mediate a cell response including activation, proliferation, differentiation, cytokine secretion, and others.

"Intracellular signaling domain" as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen-independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRγ, CD3γ, CD3δ, CD3ε, CD5, CD22, CD79a, CD79b, and CD66d.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

"Claudin 18.2-positive" cancer, including a "Claudin 18.2-positive" cancerous disease, is one comprising cells, which have Claudin 18.2 present at their cell surface. The term "Claudin 18.2-positive" also refers to a cancer that produces sufficient levels of Claudin 18.2 at the surface of cells thereof, such that a CAR-comprising cell of the present invention has a therapeutic effect, mediated by the binding of the CAR to Claudin 18.2.

The term "epitope" refers to an antigenic determinant in a molecule, i.e., to the part in a molecule that is recognized by the immune system, for example, that is recognized by a targeting moiety, such as an antibody. An epitope of a protein such as a Claudin 18.2 preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 8, 9, 10, 1 1 , 12, 13, 14, 15, 16, 17, 18, 19, 20, 21, 22, 23, 24, or 25 amino acids in length. Preferably, the epitope comprises or consists of SEQ ID NO: 1.

"Combination therapy", "in combination with" or "in conjunction with" as used herein denotes any form of concurrent, parallel, simultaneous, sequential or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations or unit dosage forms) or separately (e.g., on the same day or on different days and in any order as according to an appropriate dosing protocol for the separate compositions, formulations or unit dosage forms) in a manner and dosing regimen prescribed by a medical caretaker or according to a regulatory agency. In general, each treatment modality will be administered at a dose and/or on a time schedule determined for that treatment modality. Optionally, three or more modalities may be used in a combination therapy. Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

"Administering" or "administration of" a medicament to a patient (and grammatical equivalents of this phrase) refers to direct administration, which may be administration to a patient by a medical professional or may be self-administration, and/or indirect administration, which may be the act of prescribing a drug. E.g., a physician who instructs a patient to self-administer a medicament or provides a patient with a prescription for a drug is administering the drug to the patient.

A "complete response" or "complete remission" or "CR" indicates disappearance of all target lesions as defined in the RECIST v1.1 guideline. This does not always mean the cancer has been cured.

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats T-ALL, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a Claudin 18.2-positive cancer.

"Infusion" or "infusing" refers to the introduction of a therapeutic agent-containing solution into the body through a vein for therapeutic purposes. Generally, this is achieved via an intravenous bag.

"Overall Survival" (OS) refers to the time from patient enrollment to death or censored at the date last known alive. OS includes a prolongation in life expectancy as compared to naïve or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, may also be included in a pharmaceutical composition described herein, provided that they do not adversely affect the desired characteristics of the pharmaceutical composition.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials.

The term "respond favorably" generally refers to causing a beneficial state in a subject. With respect to cancer treatment, the term refers to providing a therapeutic effect on the subject. Positive therapeutic effects in cancer can be measured in a number of ways (See, Weber, 2009. J Nucl Med. 50 Suppl 1:1S-10S). For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

The term "sequence identity" refers to a percentage value obtained when two sequences are compared using a pairwise sequence alignment tool. In the present case, the sequence identity is preferably obtained using a local alignment tool, preferably a Basic Local Alignment Search Tool (BLAST) tool.

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrollment to disease progression. TTP is generally measured using the RECIST v1.1 criteria.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

### Detailed description of the invention

### Claudin 18.2 Targeting moiety

In **a first aspect**, the present invention relates to a Claudin 18.2 targeting moiety, also called herein as the "Claudin 18.2 targeting moiety of the invention", that is capable of binding to the amino acid sequence of SEQ ID NO: 1. By "capable of binding to the amino acid sequence of SEQ ID NO: 1" is meant that the Claudin 18.2 targeting moiety has binding affinity for SEQ ID NO: 1. Preferably, the binding affinity is specific, i.e., the Claudin 18.2 targeting moiety is capable of specifically binding to SEQ ID NO: 1 (which is its target molecule) in a manner which distinguishes from the binding to non-target molecules, i.e., from other molecules that are not SEQ ID NO: 1. Thus, the Claudin 18.2 targeting moiety either does not bind to non-target molecules or exhibits negligible or substantially-reduced (as compared to the target) e. g. background, binding to nontarget molecules. The Claudin 18.2 targeting moiety specifically recognises SEQ ID NO: 1. Specific binding between the Claudin 18.2 targeting moiety and the SEQ ID NO: 1 can be assessed by numerous techniques, such as surface plasmon resonance, flow cytometry, or ELISA assays.

In some embodiments of the first aspect, the Claudin 18.2 targeting moiety is an antibody, anticalin, repebody, monobody, scFv, Fab, scFab, affibody, fynomer, DARPin, nanobody, or peptide aptamer that specifically binds to SEQ ID NO: 1. Preferably, the targeting moiety of the first aspect can be selected from the targeting moieties defined in aspects two, three and four, described in detail below.

A **second aspect** of the present invention refers to a Claudin 18.2 targeting moiety, also called herein the "**Claudin 18.2 targeting moiety CL6**", that specifically binds to the amino acid sequence of SEQ ID NO: 1, and that comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 2, or a sequence with at least 85%, 86%, 87%, 88%, 89%, 90%, 91%, 92%, 93%, 94%, 95%, 96%, 97%, 98%, 99% or 100% sequence identity to SEQ ID NO: 2;
- LCDR2 comprises, consists, or consists essentially of SEQ ID NO: 3, or a sequence with at least 85%, 86%, 87%, 88%, 89%, 90%, 91%, 92%, 93%, 94%, 95%, 96%, 97%, 98%, 99% or 100% sequence identity to SEQ ID NO: 3;
- LCDR3 comprises, consists, or consists essentially of SEQ ID NO: 4, or a sequence with at least 85%, 86%, 87%, 88%, 89%, 90%, 91%, 92%, 93%, 94%, 95%, 96%, 97%, 98%, 99% or 100% sequence identity to SEQ ID NO: 4;
- HCDR1 comprises, consists, or consists essentially of SEQ ID NO: 5, or a sequence with at least 85%, 86%, 87%, 88%, 89%, 90%, 91%, 92%, 93%, 94%, 95%, 96%, 97%, 98%, 99% or 100% sequence identity to SEQ ID NO: 5;
- HCDR2 comprises, consists, or consists essentially of SEQ ID NO: 6, or a sequence with at least 85%, 86%, 87%, 88%, 89%, 90%, 91%, 92%, 93%, 94%, 95%, 96%, 97%, 98%, 99% or 100% sequence identity to SEQ ID NO: 6; and
- HCDR3 comprises, consists, or consists essentially of SEQ ID NO: 7, or a sequence with at least 85%, 86%, 87%, 88%, 89%, 90%, 91%, 92%, 93%, 94%, 95%, 96%, 97%, 98%, 99% or 100% sequence identity to SEQ ID NO: 7.

In some embodiments of the second aspect, the Claudin 18.2 targeting moiety CL6 is an antibody, F(ab')2, Fab, preferably single chain variable fragment (scFv), or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 2, LCDR2 consists of SEQ ID NO: 3, LCDR3 consists of SEQ ID NO: 4, HCDR1 consists of SEQ ID NO: 5, HCDR2 consists of SEQ ID NO: 6, and HCDR3 consists of SEQ ID NO: 7.

Preferably, the Claudin 18.2 targeting moiety **CL6** of the second aspect is an antibody, F(ab')2, Fab, preferably scFv, or scFab comprising a VL domain and a VH domain, wherein the VL domain comprises, consists, or consists essentially of SEQ ID NO: 8, or a sequence with at least 85%, 86%, 87%, 88%, 89%, 90%, 91%, 92%, 93%, 94%, 95%, 96%, 97%, 98%, 99% or 100% sequence identity to SEQ ID NO: 8; and wherein the VH domain comprises, consists, or consists essentially of SEQ ID NO: 9, or a sequence with at least 85%, 86%, 87%, 88%, 89%, 90%, 91%, 92%, 93%, 94%, 95%, 96%, 97%, 98%, 99% or 100% sequence identity to SEQ ID NO: 9. In some embodiments, the Claudin 18.2 targeting moiety **CL6** is an antibody, scFv, Fab, or scFab comprising a VL domain and VH domain, wherein the VL domain comprises SEQ ID NO: 8 and the VH domain comprises SEQ ID NO: 9. In some embodiments, the Claudin 18.2 targeting moiety **CL6** is an antibody, scFv, Fab, or scFab comprising a VL domain and VH domain, wherein the VL domain consists of SEQ ID NO: 8 and the VH domain consists of SEQ ID NO: 9.

The VL and VH of the Claudin 18.2 targeting moiety **CL6** of the second aspect, or any of its embodiments, may be humanized. In the humanization process, the framework regions of the variable region of a targeting moiety may be modified, whereas the CDRs are maintained constant. By "maintained constant" it is referred that, while the framework regions of the Claudin 18.2 targeting moiety can vary (for instance, they can be different depending on whether it is a murine ScFv or a humanized ScFv), the sequences belonging to the CDRs are not changed or are invariable.

In the context of the present invention and in the context of the embodiments that refer to a percentage of identity over a sequence (e.g. VH or VL sequence) where some regions of said sequences (e.g., the CDRs) are maintained constant, the percentage of identity is obtained by aligning the sequences (e.g. VH or VL sequence), preferably using BLAST tool (or a similar local alignment algorithm) and, once the percentage of identity is obtained, it is checked (by, e.g. by visual inspection) whether the CDRs are maintained constant between both sequences. In other words, both sequences are aligned and only the mismatches in the framework regions, and not in the CDRs, are considered to obtain the % of sequence identity between both sequences.

Therefore, in some embodiments of the second aspect, Claudin 18.2 targeting moiety CL6 is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 8, wherein the CDRs comprised in said VL are maintained constant (i.e., non-variable) and consists of SEQ ID NOs: 2, 3, and 4; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 9, wherein the CDRs comprised in said VH are maintained constant and consist of SEQ ID NOs: 5, 6, and 7. Preferably, the Claudin 18.2 targeting moiety **CL6** is a scFv comprising a VL and a VH domains, wherein the VL domain comprises a sequence having at least 85% sequence identity to SEQ ID NO: 8, wherein the CDRs comprised in said VL consists of SEQ ID NOs: 2, 3, and 4; and wherein the VH domain comprises a sequence having at least 85% sequence identity to SEQ ID NO: 9, wherein the CDRs comprised in said VH consist of SEQ ID NOs: 5, 6, and 7.

Thus, in an embodiment of the second aspect, Claudin 18.2 targeting moiety **CL6** is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 8, and wherein said VL domain comprises CDR regions consisting of LCDR1 as set forth in SEQ ID NO: 2, LCDR2 as set forth in SEQ ID NO: 3, LCDR3 as set forth in SEQ ID NO: 4, and
- said VH domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 9, and wherein said VH domain comprises CDR regions consisting of HCDR1 as set forth in SEQ ID NO: 5, HCDR2 as set forth in SEQ ID NO: 6, and HCDR3 as set forth in SEQ ID NO: 7 and
wherein the Claudin 18.2 targeting moiety **CL6** is capable of binding to SEQ ID NO: 1, preferably as measured by Enzyme linked immunosorbent assay (ELISA).

In an embodiment of the second aspect, the Claudin 18.2 targeting moiety **CL6** is an antibody, F(ab')2, Fab, preferably scFv, or scFab, and is capable of binding to SEQ ID NO: 1 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 8, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 9, and
wherein the LCDR1, 2, and 3 comprised in the VL domain consist of SEQ ID NO: 2, 3 or 4, respectively, and wherein the HCDR1, 2 and 3 comprised in said VH domain consists of SEQ ID NO: 5, 6 or 7, respectively.

Preferably, the Claudin 18.2 targeting moiety **CL6** comprises, consists, or consists essentially of SEQ ID NO: 44 or 12, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 44 or 12. Most preferably, the Claudin 18.2 targeting moiety **CL6** comprises SEQ ID NO: 44 or 12. Most preferably, the Claudin 18.2 targeting moiety **CL6** consists of SEQ ID NO: 44 or 12.

A **third aspect** of the present invention refers to a Claudin 18.2 targeting moiety, also called herein the "Claudin 18.2 targeting moiety CL3", that specifically binds to the amino acid sequence of SEQ ID NO: 1, and that comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 14, or a sequence with at least 85%, 86%, 87%, 88%, 89%, 90%, 91%, 92%, 93%, 94%, 95%, 96%, 97%, 98%, 99% or 100% sequence identity to SEQ ID NO: 14;
- LCDR2 comprises, consists, or consists essentially of SEQ ID NO: 15, or a sequence with at least 85%, 86%, 87%, 88%, 89%, 90%, 91%, 92%, 93%, 94%, 95%, 96%, 97%, 98%, 99% or 100% sequence identity to SEQ ID NO: 15;
- LCDR3 comprises, consists, or consists essentially of SEQ ID NO: 16, or a sequence with at least 85%, 86%, 87%, 88%, 89%, 90%, 91%, 92%, 93%, 94%, 95%, 96%, 97%, 98%, 99% or 100% sequence identity to SEQ ID NO: 16;
- HCDR1 comprises, consists, or consists essentially of SEQ ID NO: 17, or a sequence with at least 85%, 86%, 87%, 88%, 89%, 90%, 91%, 92%, 93%, 94%, 95%, 96%, 97%, 98%, 99% or 100% sequence identity to SEQ ID NO: 17;
- HCDR2 comprises, consists, or consists essentially of SEQ ID NO: 18, or a sequence with at least 85%, 86%, 87%, 88%, 89%, 90%, 91%, 92%, 93%, 94%, 95%, 96%, 97%, 98%, 99% or 100% sequence identity to SEQ ID NO: 18; and
- HCDR3 comprises, consists, or consists essentially of SEQ ID NO: 19, or a sequence with at least 85%, 86%, 87%, 88%, 89%, 90%, 91%, 92%, 93%, 94%, 95%, 96%, 97%, 98%, 99% or 100% sequence identity to SEQ ID NO: 19.

In some embodiments of the third aspect, the Claudin 18.2 targeting moiety **CL3** is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19.

Preferably, the Claudin 18.2 targeting moiety **CL3** of the third aspect is an antibody, F(ab')2, Fab, preferably scFv, or scFab comprising a VL domain and a VH domain, wherein the VL domain comprises, consists, or consists essentially of SEQ ID NO: 20, or a sequence with at least 85%, 86%, 87%, 88%, 89%, 90%, 91%, 92%, 93%, 94%, 95%, 96%, 97%, 98%, 99% or 100% sequence identity to SEQ ID NO: 20; and wherein the VH domain comprises, consists, or consists essentially of SEQ ID NO: 21, or a sequence with at least 85%, 86%, 87%, 88%, 89%, 90%, 91%, 92%, 93%, 94%, 95%, 96%, 97%, 98%, 99% or 100% sequence identity to SEQ ID NO: 21. In some embodiments, the Claudin 18.2 targeting moiety **CL3** is an antibody, scFv, Fab, or scFab comprising a VL domain and VH domain, wherein the VL domain comprises SEQ ID NO: 20 and the VH domain comprises SEQ ID NO: 21. In some embodiments, the Claudin 18.2 targeting moiety **CL3** is an antibody, scFv, Fab, or scFab comprising a VL domain and VH domain, wherein the VL domain consists of SEQ ID NO: 20 and the VH domain consists of SEQ ID NO: 21.

Similarly, as explained for the Claudin 18.2 targeting moiety CL6 above, the VL and VH of the Claudin 18.2 targeting moiety **CL3** of the third aspect, or any of its embodiments, may be humanized. Therefore, in some embodiments of the third aspect, Claudin 18.2 targeting moiety **CL3** is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 20, wherein the CDRs comprised in said VL are maintained constant (i.e., non-variable) and consists of SEQ ID NOs: 14, 15, and 16; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 21, wherein the CDRs comprised in said VH are maintained constant and consist of SEQ ID NOs: 17, 18, and 19. Preferably, the Claudin 18.2 targeting moiety **CL3** is a scFv comprising a VL and a VH domains, wherein the VL domain comprises a sequence having at least 85% sequence identity to SEQ ID NO: 20, wherein the CDRs comprised in said VL consists of SEQ ID NOs: 14, 15, and 16; and wherein the VH domain comprises a sequence having at least 85% sequence identity to SEQ ID NO: 21, wherein the CDRs comprised in said VH consist of SEQ ID NOs: 17, 18, and 19.

Thus, in an embodiment of the third aspect, Claudin 18.2 targeting moiety CL3 is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 20, and wherein said VL domain comprises CDR regions consisting of LCDR1 as set forth in SEQ ID NO: 14, LCDR2 as set forth in SEQ ID NO: 15, LCDR3 as set forth in SEQ ID NO: 16, and
- said VH domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 21, and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19 and
wherein the Claudin 18.2 targeting moiety **CL3** is capable of binding to SEQ ID NO: 1, preferably as measured by Enzyme linked immunosorbent assay (ELISA).

In an embodiment of the third aspect, the Claudin 18.2 targeting moiety **CL3** is an antibody, F(ab')2, Fab, preferably scFv, or scFab, and is capable of binding to SEQ ID NO: 1 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 20, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 21, and
wherein the LCDR1, 2, and 3 comprised in the VL domain consist of SEQ ID NO: 14, 15 and 16, respectively, and wherein the HCDR1, 2 and 3 comprised in said VH domain consists of SEQ ID NO: 17, 18 and 19, respectively.

Preferably, the Claudin 18.2 targeting moiety **CL3** comprises, consists, or consists essentially of SEQ ID NO: 46 or 24, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 46 or 24. Most preferably, the Claudin 18.2 targeting moiety **CL3** comprises SEQ ID NO: 46 or 24. Most preferably, the Claudin 18.2 targeting moiety **CL3** consists of SEQ ID NO: 46 or 24.

A **fourth aspect** of the present invention refers to a Claudin 18.2 targeting moiety, also called herein the "Claudin 18.2 targeting moiety **CL2**", that specifically binds to the amino acid sequence of SEQ ID NO: 1, and that comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 26, or a sequence with at least 85%, 86%, 87%, 88%, 89%, 90%, 91%, 92%, 93%, 94%, 95%, 96%, 97%, 98%, 99% or 100% sequence identity to SEQ ID NO: 26;
- LCDR2 comprises, consists, or consists essentially of SEQ ID NO: 27, or a sequence with at least 85%, 86%, 87%, 88%, 89%, 90%, 91%, 92%, 93%, 94%, 95%, 96%, 97%, 98%, 99% or 100% sequence identity to SEQ ID NO: 27;
- LCDR3 comprises, consists, or consists essentially of SEQ ID NO: 28, or a sequence with at least 85%, 86%, 87%, 88%, 89%, 90%, 91%, 92%, 93%, 94%, 95%, 96%, 97%, 98%, 99% or 100% sequence identity to SEQ ID NO: 28;
- HCDR1 comprises, consists, or consists essentially of SEQ ID NO: 29, or a sequence with at least 85%, 86%, 87%, 88%, 89%, 90%, 91%, 92%, 93%, 94%, 95%, 96%, 97%, 98%, 99% or 100% sequence identity to SEQ ID NO: 29;
- HCDR2 comprises, consists, or consists essentially of SEQ ID NO: 30, or a sequence with at least 85%, 86%, 87%, 88%, 89%, 90%, 91%, 92%, 93%, 94%, 95%, 96%, 97%, 98%, 99% or 100% sequence identity to SEQ ID NO: 30; and
- HCDR3 comprises, consists, or consists essentially of SEQ ID NO: 31, or a sequence with at least 85%, 86%, 87%, 88%, 89%, 90%, 91%, 92%, 93%, 94%, 95%, 96%, 97%, 98%, 99% or 100% sequence identity to SEQ ID NO: 31.

In some embodiments of the fourth aspect, the Claudin 18.2 targeting moiety **CL2** is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 26, LCDR2 consists of SEQ ID NO: 27, LCDR3 consists of SEQ ID NO: 28, HCDR1 consists of SEQ ID NO: 29, HCDR2 consists of SEQ ID NO: 30, and HCDR3 consists of SEQ ID NO: 31.

Preferably, the Claudin 18.2 targeting moiety **CL2** of the fourth aspect is an antibody, F(ab')2, Fab, preferably scFv, or scFab comprising a VL domain and a VH domain, wherein the VL domain comprises, consists, or consists essentially of SEQ ID NO: 32, or a sequence with at least 85%, 86%, 87%, 88%, 89%, 90%, 91%, 92%, 93%, 94%, 95%, 96%, 97%, 98%, 99% or 100% sequence identity to SEQ ID NO: 32; and wherein the VH domain comprises, consists, or consists essentially of SEQ ID NO: 33, or a sequence with at least 85%, 86%, 87%, 88%, 89%, 90%, 91%, 92%, 93%, 94%, 95%, 96%, 97%, 98%, 99% or 100% sequence identity to SEQ ID NO: 33. In some embodiments, the Claudin 18.2 targeting moiety **CL2** is an antibody, scFv, Fab, or scFab comprising a VL domain and VH domain, wherein the VL domain comprises SEQ ID NO: 32 and the VH domain comprises SEQ ID NO: 33. In some embodiments, the Claudin 18.2 targeting moiety **CL2** is an antibody, scFv, Fab, or scFab comprising a VL domain and VH domain, wherein the VL domain consists of SEQ ID NO: 32 and the VH domain consists of SEQ ID NO: 33.

Similarly, as explained for the Claudin 18.2 targeting moiety CL6 above, the VL and VH of the Claudin 18.2 targeting moiety **CL2** of the fourth aspect, or any of its embodiments, may be humanized. Therefore, in some embodiments of the fourth aspect, Claudin 18.2 targeting moiety **CL2** is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 32, wherein the CDRs comprised in said VL are maintained constant (i.e., non-variable) and consists of SEQ ID NOs: 26, 27 and 28; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 33, wherein the CDRs comprised in said VH are maintained constant and consist of SEQ ID NOs: 29, 30 and 31. Preferably, the Claudin 18.2 targeting moiety **CL2** is a scFv comprising a VL and a VH domains, wherein the VL domain comprises a sequence having at least 85% sequence identity to SEQ ID NO: 32, wherein the CDRs comprised in said VL consists of SEQ ID NOs: 26, 27 and 28; and wherein the VH domain comprises a sequence having at least 85% sequence identity to SEQ ID NO: 33, wherein the CDRs comprised in said VH consist of SEQ ID NOs: 29, 30 and 31.

Thus, in an embodiment of the fourth aspect, Claudin 18.2 targeting moiety **CL2** is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 32, and wherein said VL domain comprises CDR regions consisting of LCDR1 as set forth in SEQ ID NO: 26, LCDR2 as set forth in SEQ ID NO: 27, LCDR3 as set forth in SEQ ID NO: 28, and
- said VH domain comprises a sequence with at least 80%, 81%, 82%, 83%, 84%, 85%, 86%, 87%, 88%,, 89%, 90%, 91%, 92%, 93%, 94%, 95%, 96%, 97%, 98%, or 99% sequence identity to SEQ ID NO: 33, and wherein said VH domain comprises CDR regions consisting of HCDR1 as set forth in SEQ ID NO: 29, HCDR2 as set forth in SEQ ID NO: 30, and HCDR3 as set forth in SEQ ID NO: 31 and
wherein the Claudin 18.2 targeting moiety **CL2** is capable of binding to SEQ ID NO: 1, preferably as measured by-Enzyme linked immunosorbent assay (ELISA).

In an embodiment of the fourth aspect, the Claudin 18.2 targeting moiety **CL2** is an antibody, F(ab')2, Fab, preferably scFv, or scFab, and is capable of binding to SEQ ID NO: 1 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 32, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 33, and
wherein the LCDR1, 2, and 3 comprised in the VL domain consist of SEQ ID NO: 26, 27 and 28, respectively, and wherein the HCDR1, 2 and 3 comprised in said VH domain consists of SEQ ID NO: 29, 30 and 31, respectively.

Preferably, the Claudin 18.2 targeting moiety **CL2** comprises, consists, or consists essentially of SEQ ID NO: 36 or 48, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 36 or 48. Most preferably, the Claudin 18.2 targeting moiety **CL2** comprises SEQ ID NO: 36 or 48. Most preferably, the Claudin 18.2 targeting moiety **CL2** consists of SEQ ID NO: 36 or 48.

In a preferred embodiment of the first, second, third and fourth aspects, the VL and the VH domains comprised in the Claudin 18.2 targeting moieties are linked by a peptide linker. In some embodiments, the linker comprises at least 5 amino acids, preferably between 5 and 25, preferably between 10-20 amino acids, most preferably 20 amino acids. Preferably, the amino acid is G and/or S. Preferably, the peptide linker comprises, consists or consists essentially of SEQ ID NO: 39, or a sequence that has at least 85%, 86%, 87%, 88%, 89%, 90%, 91%, 92%, 93%, 94%, 95%, 96%, 97%, 98%, 99% or 100% sequence identity to SEQ ID NO: 39.

In a preferred embodiment of the first, second, third and fourth aspects, the Claudin 18.2 targeting moieties further comprises a signal peptide that is placed preferably at the N-terminal region of the Claudin 18.2 targeting moiety. Preferably, the signal peptide comprises, consists or consists essentially of SEQ ID NO: 38, or a sequence that has at least 85%, 86%, 87%, 88%, 89%, 90%, 91%, 92%, 93%, 94%, 95%, 96%, 97%, 98%, 99% or 100% sequence identity to SEQ ID NO: 38.

In an embodiment, the Claudin 18.2 targeting moieties of the first to fourth aspects is comprised in a chimeric antigen receptor (CAR), as will be further defined below:

### CAR anti-Claudin 18.2 of the invention

The Claudin 18.2 targeting moieties as defined above in any of the previous aspects, or any of their embodiments, can be part of a chimeric antigen receptor (CAR), also called herein "the CAR anti-claudin 18.2 of the invention". Thus, in a preferred embodiment of the first, second, third and fourth aspects, the present invention provides a CAR comprising:
a) an extracellular domain comprising a Claudin 18.2 targeting moiety as defined in the first, second, third and fourth aspects or in any of the embodiments disclosed above,
b) a transmembrane domain; and
c) an intracellular signaling domain.

Preferably, the CAR further comprises other elements, such as a leader peptide and a hinge region between a) and b).

Each of the elements of the CAR according to this embodiment of the first to fourth aspects are further developed below:
*a)* *extracellular domain comprising a Claudin 18.2 targeting moiety* as *defined in the first, second, third, and fourth aspects or any of its embodiments.*

In an embodiment the extracellular domain comprises at least one, preferably one, of the Claudin 18.2 targeting moieties as defined above in the first, second, third, or fourth aspects, or any of their embodiments. Thus, all the embodiments defined above in said aspects apply herein.

### b) transmembrane domain

The transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, CD154 or ICOS.

A transmembrane domain may be synthetic or a variant of a naturally occurring transmembrane domain. In some embodiments, synthetic or variant transmembrane domains comprise predominantly hydrophobic residues such as leucine and valine.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, ICOS or a variant thereof, wherein the variant thereof has a 95%, 98%, or 99% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, or CD154.

In some embodiments, the transmembrane domain comprises the transmembrane domain of CD8 or a variant thereof, wherein the variant thereof has a 95% sequence identity.

In some embodiments, the transmembrane domain comprises the **transmembrane domain** of CD8 or a variant thereof. Preferably, the transmembrane domain consists or consists essentially of SEQ ID NO: 41, or a sequence that has at least 85%, 86%, 87%, 88%, 89%, 90%, 91%, 92%, 93%, 94%, 95%, 96%, 97%, 98%, 99% or 100% sequence identity to SEQ ID NO: 41.

In an embodiment, the transmembrane domain is linked to a **hinge domain**, wherein the hinge domain is preferably placed at the N-terminal of the transmembrane domain. Preferably, the hinge domain consists or consists essentially of SEQ ID NO: 40, or a sequence that has at least 85%, 86%, 87%, 88%, 89%, 90%, 91%, 92%, 93%, 94%, 95%, 96%, 97%, 98%, 99% or 100% sequence identity to SEQ ID NO: 40.

### c) Intracellular signaling domain

The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In some embodiments, the intracellular signaling domain comprises the intracellular domain of CD3ζ, FcRγ, CD3γ, CD3δ, CD3ε, CD5, CD22, CD79a, CD79b, CD66b, or a variant thereof, wherein the variant thereof has a 95%, 98% or 99% sequence identity. In some embodiments, the intracellular signaling domain comprises the intracellular domain of CD3ζ, FcRγ, CD3γ, CD3δ, CD3ε, CD5, CD22, CD79a, CD79b or CD66b.

In some embodiments, the intracellular signaling domain comprises the intracellular domain of CD3ζ or a variant thereof, wherein the variant thereof has a 95%, 98%, or 99% sequence identity.

In a preferred embodiment, the intracellular signalling domain consists or consists essentially of SEQ ID NO: 43, or a sequence that has at least 85%, 86%, 87%, 88%, 89% 90%, 91%, 92%, 93%, 94%, 95%, 96%, 97%, 98%, 99% or 100% sequence identity to SEQ ID NO: 43. In some embodiments, the intracellular signaling domain comprises SEQ ID NO: 43. In some embodiments, the intracellular signaling domain consists of SEQ ID NO: 43.

Optionally, at least a costimulatory signaling domain may also be present in the CAR according to the first, second, third, or fourth aspects or any of its embodiments:

### d) Costimulatory signaling domain

In some embodiments, the CAR may further comprise a costimulatory signaling domain. In

some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276, ICOS or a variant thereof, wherein the variant thereof has a 95%, 98%, or 99% sequence identity.

In some embodiments, the costimulatory signaling domain comprises the intracellular domain of CD27, CD28, CD137 or 4-1BB, CD134, CD30, CD40, lymphocyte function-associated antigen-1 (LFA-1), CD2, CD7, LIGHT, NKG2C, CD276 or ICOS.

In some embodiments, the costimulatory signaling domain comprises the intracellular domain of CD137 or a variant thereof, wherein the variant thereof has a 95%, 98% or 99% sequence identity. In some embodiments, the costimulatory signaling domain comprises the intracellular domain of CD137 or 4-1BB.

In a preferred embodiment, the costimulatory signaling domain consists or consists essentially of SEQ ID NO: 42, or a sequence that has at least 85%, 86%, 87%, 88%, 89% 90%, 91%, 92%, 93%, 94%, 95%, 96%, 97%, 98%, 99% or 100% sequence identity to SEQ ID NO: 42. In some embodiments, the costimulatory signaling domain comprises SEQ ID NO: 42. In some embodiments, the costimulatory signaling domain consists of SEQ ID NO: 42.

### Full CARs sequence according to the first to fourth aspects of the present invention

In some embodiments, the CAR comprises:
(a) an extracellular domain comprising a Claudin 18.2 targeting moiety that is capable of specifically binding to SEQ ID NO: 1; and
(b) a transmembrane domain, preferably the transmembrane domain of CD8;
(c) an intracellular signaling domain, preferably the intracellular domain of CD3ζ; and
(d) at least a costimulatory signaling domain, preferably the intracellular domain of CD137.

In some embodiments, the CAR comprises:
(a) an extracellular domain comprising a Claudin 18.2 targeting moiety that is a:
   - scFv **(CL6)** comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 2, LCDR2 consists of SEQ ID NO: 3, LCDR3 consists of SEQ ID NO: 4, HCDR1 consists of SEQ ID NO: 5, HCDR2 consists of SEQ ID NO: 6, and HCDR3 consists of SEQ ID NO: 7;
   - scFv **(CL3)** comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19, or
   - scFv **(CL2)** comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 26, LCDR2 consists of SEQ ID NO: 27, LCDR3 consists of SEQ ID NO: 28, HCDR1 consists of SEQ ID NO: 29, HCDR2 consists of SEQ ID NO: 30, and HCDR3 consists of SEQ ID NO: 31; and
(b) a transmembrane domain, preferably the transmembrane domain of CD8, more preferably comprising SEQ ID NO: 41;
(c) an intracellular signaling domain, preferably the intracellular domain of CD3ζ; more preferably comprising SEQ ID NO: 43; and
(d) at least a costimulatory signaling domain, preferably the intracellular domain of CD137 more preferably comprising SEQ ID NO: 42.

In some embodiments, the CAR comprises:
(a) an extracellular domain comprising a Claudin 18.2 targeting moiety that is a:
   - scFv **(CL6)** comprising a VL domain and VH domain, wherein the VL domain comprises a sequence with at least 85%, preferably 95%, sequence identity to SEQ ID NO: 8 and wherein said VL domain comprises CDR regions consisting of LCDR1 as set forth in SEQ ID NO: 2, LCDR2 as set forth in SEQ ID NO: 3, LCDR3 as set forth in SEQ ID NO: 4; and wherein the VH domain comprises a sequence with at least 85% sequence identity to SEQ ID NO: 9, and wherein said VH domain comprises CDR regions consisting of HCDR1 as set forth in SEQ ID NO: 5, HCDR2 as set forth in SEQ ID NO: 6, and HCDR3 as set forth in SEQ ID NO: 7;
   - scFv **(CL3)** comprising a VL domain and VH domain, wherein the VL domain comprises a sequence with at least 85%, preferably 95%, sequence identity to SEQ ID NO: 20 and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21, and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19; or
   - scFv **(CL2)** comprising a VL domain and VH domain, wherein the VL domain comprises a sequence with at least 85%, preferably 95%, sequence identity to SEQ ID NO: 32 and wherein said VL domain comprises CDR regions consisting of LCDR1 as set forth in SEQ ID NO: 26, LCDR2 as set forth in SEQ ID NO: 27, LCDR3 as set forth in SEQ ID NO: 28; and wherein the VH domain comprises a sequence with at least 85% sequence identity to SEQ ID NO: 33, and wherein said VH domain comprises CDR regions consisting of HCDR1 as set forth in SEQ ID NO: 29, HCDR2 as set forth in SEQ ID NO: 30, and HCDR3 as set forth in SEQ ID NO: 31; and
(b) a transmembrane domain, preferably the transmembrane domain of CD8, more preferably comprising SEQ ID NO: 41;
(c) an intracellular signaling domain, preferably the intracellular domain of CD3ζ; more preferably comprising SEQ ID NO: 43; and
(d) at least a costimulatory signaling domain, preferably the intracellular domain of CD137 more preferably comprising SEQ ID NO: 42.

In some embodiments, the CAR comprises:
(a) an extracellular domain comprising a Claudin 18.2 targeting moiety that is a:
   - scFv **(CL6)**comprising a VL domain and VH domain, wherein the VL domain comprises SEQ ID NO: 8, and the VH domain comprises SEQ ID NO: 9,
   - scFv **(CL3)**comprising a VL domain and VH domain, wherein the VL domain comprises SEQ ID NO: 20, and the VH domain comprises SEQ ID NO: 21,
   - scFv **(CL2)** comprising a VL domain and VH domain, wherein the VL domain comprises SEQ ID NO: 32, and the VH domain comprises SEQ ID NO: 33, and
(b) a transmembrane domain, preferably the transmembrane domain of CD8, more preferably comprising SEQ ID NO: 41;
(c) an intracellular signaling domain, preferably the intracellular domain of CD3ζ; more preferably comprising SEQ ID NO: 43; and
(d) at least a costimulatory signaling domain, preferably the intracellular domain of CD137 more preferably comprising SEQ ID NO: 42.

In some embodiments, the CAR comprises:
(a) an extracellular domain comprising a Claudin 18.2 targeting moiety that is a:
   - scFv **(CL6)** comprising a VL domain and VH domain, wherein the VL domain consists of SEQ ID NO: 8, and the VH domain consist of SEQ ID NO: 9,
   - scFv **(CL3)** comprising a VL domain and VH domain, wherein the VL domain consists of SEQ ID NO: 20, and the VH domain consist of SEQ ID NO: 21,
   - scFv **(CL2)** comprising a VL domain and VH domain, wherein the VL domain consist of SEQ ID NO: 32, and the VH domain consist of SEQ ID NO: 33,and
(b) a transmembrane domain, preferably the transmembrane domain of CD8, more preferably comprising SEQ ID NO: 41;
(c) an intracellular signaling domain, preferably the intracellular domain of CD3ζ; more preferably comprising SEQ ID NO: 43; and
(d) at least a costimulatory signaling domain, preferably the intracellular domain of CD137 more preferably comprising SEQ ID NO: 42.

In some embodiments, the CAR comprises:
(a) an extracellular domain comprising a Claudin 18.2 targeting moiety that is a scFv comprising SEQ ID NOs: 44, 12, 46, 24, 36, or 48;
(b) a transmembrane domain, preferably the transmembrane domain of CD8, more preferably comprising SEQ ID NO: 41;
(c) an intracellular signaling domain, preferably the intracellular domain of CD3ζ; more preferably comprising SEQ ID NO: 43; and
(d) at least a costimulatory signaling domain, preferably the intracellular domain of CD137 more preferably comprising SEQ ID NO: 42.

In some embodiments, the CAR comprises or consists of SEQ ID NO: 13 **(CAR CL6)**, or a sequence that has at least 85%, 86%, 87%, 88%, 89% 90%, 91%, 92%, 93%, 94%, 95%, 96%, 97%, 98%, 99% or 100% sequence identity to SEQ ID NO: 13. In some embodiments, the CAR comprises SEQ ID NO: 13. In some embodiments, the CAR consists of SEQ ID NO: 13.

In some embodiments, the CAR comprises or consists of SEQ ID NO: 45 **(CAR CL6)**, or a sequence that has at least 85%, 86%, 87%, 88%, 89% 90%, 91%, 92%, 93%, 94%, 95%, 96%, 97%, 98%, 99% or 100% sequence identity to SEQ ID NO: 45. In some embodiments, the CAR comprises SEQ ID NO: 45. In some embodiments, the CAR consists of SEQ ID NO: 45.

In some embodiments, the CAR comprises or consists of SEQ ID NO: 25 **(CAR CL3)**, or a sequence that has at least 85%, 86%, 87%, 88%, 89% 90%, 91%, 92%, 93%, 94%, 95%, 96%, 97%, 98%, 99% or 100% sequence identity to SEQ ID NO: 25. In some embodiments, the CAR comprises SEQ ID NO: 25. In some embodiments, the CAR consists of SEQ ID NO: 25.

In some embodiments, the CAR comprises or consists of SEQ ID NO: 47 **(CAR CL3)**, or a sequence that has at least 85%, 86%, 87%, 88%, 89% 90%, 91%, 92%, 93%, 94%, 95%, 96%, 97%, 98%, 99% or 100% sequence identity to SEQ ID NO: 47. In some embodiments, the CAR comprises SEQ ID NO: 47. In some embodiments, the CAR consists of SEQ ID NO: 47.

In some embodiments, the CAR comprises or consists of SEQ ID NO: 37 **(CAR CL2)**, or a sequence that has at least 85%, 86%, 87%, 88%, 89% 90%, 91%, 92%, 93%, 94%, 95%, 96%, 97%, 98%, 99% or 100% sequence identity to SEQ ID NO: 37. In some embodiments, the CAR comprises SEQ ID NO: 37. In some embodiments, the CAR consists of SEQ ID NO: 37.

In some embodiments, the CAR comprises or consists of SEQ ID NO: 49 **(CAR CL2)**, or a sequence that has at least 85%, 86%, 87%, 88%, 89% 90%, 91%, 92%, 93%, 94%, 95%, 96%, 97%, 98%, 99% or 100% sequence identity to SEQ ID NO: 49. In some embodiments, the CAR comprises SEQ ID NO: 49. In some embodiments, the CAR consists of SEQ ID NO: 49.

### Nucleic acid molecules of the invention

In **a fifth aspect**, the present invention provides a nucleic acid, also called herein "the nucleic acid of the invention", encoding one or more of the Claudin 18.2 targeting moieties defined above in the first, second, third or fourth aspects, including any one of the CAR anti-Claudin 18.2 of the invention as defined above, or any of their embodiments. The nucleic acid sequence may encode the chimeric receptor links together a number of modular components that can be excised and replaced with other components in order to customize the chimeric receptor for efficient T cell activation and recognition of Claudin 18.2.

In a preferred embodiment, the nucleic acid comprises, consists or consists essentially of SEQ ID NO: 10 and/or 11 (VL and VH CL6, respectively), or a sequence that has at least 85%, 86%, 87%, 88%, 89% 90%, 91%, 92%, 93%, 94%, 95%, 96%, 97%, 98%, 99% or 100% sequence identity to SEQ ID NO: 10 and/or 11, respectively.

In a preferred embodiment, the nucleic acid comprises, consists or consists essentially of SEQ ID NO: 22 and/or 23 (VL and VH **CL3**, respectively), or a sequence that has at least 85%, 86%, 87%, 88%, 89% 90%, 91%, 92%, 93%, 94%, 95%, 96%, 97%, 98%, 99% or 100% sequence identity to SEQ ID NO: 22 and/or 23, respectively.

In a preferred embodiment, the nucleic acid comprises, consists or consists essentially of SEQ ID NO: 34 and/or 35 (VL and VH **CL2**, respectively), or a sequence that has at least 85%, 86%, 87%, 88%, 89% 90%, 91%, 92%, 93%, 94%, 95%, 96%, 97%, 98%, 99% or 100% sequence identity to SEQ ID NO: 34 and/or 35, respectively.

### Cells of the invention

In a **sixth aspect**, the invention provides a cell or a plurality of cells, also called herein "the cell or cells of the invention", comprising and/or expressing one or more of the Claudin 18.2 targeting moieties and/or the CARs, of the first, second, third and fourth aspect; and/or the nucleic acid molecule, of the fifth aspect, or any of their embodiments. In some embodiments, the cell is a natural killer cell (NK), a NK cell, a T cell, a monocyte, a macrophage, or a dendritic cell.

Preferably, the cell is a T-cell. In some embodiments, the cell is a naïve T cell, memory stem T cell or central memory T cell. It is currently thought that these cells are better suited for adaptive immunotherapy. Preferably, the cell is a T cell and comprises and/or expresses the CARs defined above (also called herein CAR-T cell).

In some embodiments, the cell is an autologous T cell. The term "autologous cell" refers to a cell obtained from the same patient that is to be treated using any one of the methods of the invention.

In some embodiments, the cell is an allo-tolerant T cell. The term "allo-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art.

In some embodiments, the T cell is a CD3-positive (CD3+) T cell. In some embodiments, the T cell is a CD8+ T cell. In some instances, the cell is a human cell. In some embodiments, the cell is a lymphoid precursor, embryonic stem cell or an induced pluripotent stem cell with the capacity to differentiate into a mature T cell. In some embodiments, the cell is a CD34-positive (CD34+) cell, preferably selected from the list consisting of HPCs or cord blood cells.

### Pharmaceutical composition of the invention

In **a seventh aspect**, the invention provides a pharmaceutical composition, also called herein "the pharmaceutical composition of the invention", comprising a plurality of cells of the invention and a pharmaceutically acceptable carrier or diluent.

A pharmaceutical composition as described herein may also contain other substances. These substances include, but are not limited to, cryoprotectants, surfactants, anti-oxidants, and stabilizing agents. The term "cryoprotectant" as used herein, includes agents which provide stability to the CARTs against freezing-induced stresses. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In some embodiments, the pharmaceutical composition comprises other substances, such as cancer growth blockers, that improve the therapeutic effect of composition.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum or other human serum components.

### Methods of treatment of the present invention

In an **eighth aspect**, the invention provides a Claudin 18.2 targeting moiety and/or the CAR according to the first, second, third or fourth aspects, a nucleic acid according to the fifth aspect, a cell according to the sixth aspect, and/or a pharmaceutical composition according to the seventh aspect, for use as a medicament or in therapy.

In a preferred embodiment, the use is in a method of treating or preventing a Claudin 18.2 positive cancer, the method comprising administering the Claudin 18.2 targeting moiety and/or the CAR comprising the same, as defined in the first, second, third or fourth aspects, the nucleic acid of the fifth aspect, the cell of the sixth aspect and/or the pharmaceutical composition of the seventh aspect to a patient in need thereof. In some embodiments, the Claudin 18.2 positive is pancreatic carcinoma, esophageal gastric carcinoma, and/or gastric carcinoma. Preferably, the tumor to be treated or prevented overexpresses Claudin 18.2, preferably having SEQ ID NO: 1.

In some embodiments, the Claudin 18.2 targeting moiety, the CARs, the cells, the nucleic acid, and/or the pharmaceutical composition of the invention is administered intravenously, intraperitoneally, into the bone marrow, into the lymph node, and /or into cerebrospinal fluid.

Additionally, the combination therapies provided herein may be used in conjunction with other types of treatment. For example, other anti-cancer treatment may be selected from the group consisting of chemotherapy, immunotherapy, surgery, radiotherapy (radiation) and/or hormone therapy, amongst other treatments associated with the current standard of care for the subject. Hence, the method and uses thereof also include the administration of two (or more) different treatments that are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered. Preferably, the method comprises administering the Claudin 18.2 targeting moieties, the CARs, the nucleic acid, the cells and/or pharmaceutical composition of the invention.

In an embodiment, the Claudin 18.2 targeting moiety and/or the CAR-expressing cell described herein is administered with at least one additional therapeutic agent simultaneously, in the same or in separate compositions, or sequentially. For sequential administration, the Claudin 18.2 targeting moiety and/or the CAR-expressing cell described herein can be administered first, and the additional agent can be administered second, or the order of administration can be reversed.

Preferably, the patient in need thereof is a human, most preferably a human having or suffering from a Claudin 18.2 positive cancer, wherein the cancer is characterized in that its cells express SEQ ID NO: 1.

In some embodiments, the patient to be treated with the method of the present invention is in complete or near-complete remission after treatment with another therapy. It may be preferable desirable to decrease the tumor burden before using the methods of the present invention because since there are several alternative effector T-cells in cases of patients with highly active relapsed/refractory cancer. In some embodiments, the patient to be treated with the method of the present invention has previously been treated with another therapy which resulted in a partial response, complete response, stable disease, decrease in progressive disease, reduced time to tumor progression or any combination thereof.

In some embodiments, the method comprises further administering an immune checkpoint. In a further embodiment, the method comprises further administering an immune checkpoint inhibitor and/or an Apoptosis Proteins (IAP) inhibitor. In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely. In some embodiments, the therapeutic agent can be, but is not limited to, conventional chemotherapy including but not limited to alkylating agents, antimetabolites, anti -microtubule agents, topoisomerase inhibitors, and cytotoxic antibiotics; high- dose chemotherapy including but not limited to high-dose Melphalan chemotherapy with or without stem cell transplant; proteasome inhibitors such as, but not limited to, bortezomib, ixazomib, and carfilzomib; immunomodulatory agents (IMiDS) such as, but not limited to, thalidomide, lenalidomide, and pomalidomide; histone deacetylase (HDAC) inhibitors such as, but not limited to panobinostat; monoclonal antibodies such as, but not limited to, daratumumab, isatuximab, or elotuzumab; bispecific antibodies; and immune checkpoint inhibitors such as, but not limited to, ipilimumab, nivolumab, and pembrolizumab.

### Methods of diagnosis of the present invention

In a **ninth aspect**, the invention provides a Claudin 18.2 targeting moiety and/or the CAR according to the first, second, third or fourth aspects, for use in the diagnostic field. Preferably, the Claudin 18.2 targeting moiety and/or the CAR according to the first, second, third or fourth aspects can be used in a method for the diagnosis, in particular the early diagnosis, of a Claudin 18.2-positive cancer, preferably a cancer that expresses SEQ ID NO: 1. Preferably, the method comprises detecting a cell expressing is performed by SEQ ID NO: 1 using the targeting moieties and/or CAR described herein.

In an embodiment, the method is an *in vitro* method for the differential diagnosis of a subject suspected of suffering from gastric cancer, pancreas cancer or esophageal cancer, the method comprising the steps of:
(a) measuring the expression levels of Claudin 18.2 protein, preferably of SEQ ID NO: 1, in a biological sample obtained from the subject, thereby obtaining a protein expression profile of said sample, wherein the measurement of the expression level of Claudin 18.2 protein, preferably of SEQ ID NO: 1, is performed by contacting the biological sample with one or more of any of the Claudin 18.2-targeting moieties defined above, and
(b) identifying the subject as suffering from gastric cancer, pancreas cancer or esophageal cancer by correlating the protein expression profile identified in step (a) with reference protein expression profiles from samples obtained from subjects previously identified as suffering from gastric cancer, pancreas cancer or esophageal cancer.

In an embodiment, the correlation of the protein expression profile identified in step (a) with reference protein expression profiles is performed by a predictive model, said predictive model having been generated by training a computer with a plurality of protein expression profiles from previously identified subjects suffering from gastric cancer, pancreas cancer or esophageal cancer by machine learning on said plurality of protein expression profiles so as to obtain representative protein expression profiles associated with from gastric cancer, pancreas cancer or esophageal cancer

### Kits

In a **tenth aspect**, the present invention provides kits and their use according to any of the previous aspects. The materials described above as well as other materials can be packaged together in any suitable combination as a kit useful for performing, or aiding in the performance of, the disclosed method. It is useful if the kit components in a given kit are designed and adapted for use together in the disclosed method. Also disclosed are kits comprising one or more of the vectors disclosed herein.

### SEQUENCE LISTING

### Target Claudin 18.2:

**SEQ ID NO: 1** QWSTQDLYNNPVTAVFNYQ
   **CL6**
**SEQ ID NO: 2 (LCDR1):** QSLLNSGSQKNY
**SEQ ID NO: 3 (LCDR2):** WAS
**SEQ ID NO: 4 (LCDR3):** QNNYYYPLT
**SEQ ID NO: 5 (HCDR1):** GYAFTNYL
**SEQ ID NO: 6 (HCDR2):** INPGSGAT
**SEQ ID NO: 7 (HCDR3):** ARIYYGNSFAY
**SEQ ID NO: 8 (VL amino acid sequence):** 
DIVMTQSPSSLTVTAGEKVTMNCKSSQSLLNSGSQKNYLTWYQQKPGQPPKLLIFWASTRE
SGVPDRFTGSGSGTDFTLTISSVQAEDLAVYYCQNNYYYPLTFGAGTKLELK
**SEQ ID NO: 9 (VH amino acid sequence):** 
QVQLQQSGAELVRPGTSVKVSCKASGYAFTNYLIEWVKERPGQGLEWIGVINPGSGATNY
NEKFKGKATLTADKSSSTAYMQLSSLTSDDSAVYFCARIYYGNSFAYWGQGTLVTVSA
**SEQ ID NO: 10 (VL nucleotide sequence):** 
GACATTGTGATGACACAGTCTCCATCCTCCCTGACTGTGACAGCAGGAGAGAAGGTCAC
TATGAACTGCAAGTCCAGTCAGAGTCTGTTGAACAGTGGAAGTCAAAAGAACTATTTGA
CCTGGTACCAGCAGAAACCAGGGCAGCCTCCTAAACTGTTGATCTTCTGGGCATCCACT
 
AGGGAATCTGGGGTCCCTGATCGCTTCACAGGCAGTGGATCTGGAACAGATTTCACTCT
CACCATCAGCAGTGTGCAGGCTGAAGACCTGGCAGTTTATTACTGTCAGAATAATTATTA
TTATCCACTCACGTTCGGTGCTGGGACCAAGCTGGAGCTGAAA
**SEQ ID NO: 11 (VH nucleotide sequence):** 
CAGGTCCAGCTGCAGCAGTCTGGAGCTGAGCTGGTAAGACCTGGGACTTCAGTGAAGG
TGTCCTGCAAGGCTTCTGGATACGCCTTCACTAATTACTTGATAGAGTGGGTAAAGGAG
AGGCCTGGACAGGGCCTTGAGTGGATTGGAGTGATTAATCCTGGAAGTGGTGCTACTA
ACTACAATGAGAAGTTCAAGGGCAAGGCAACACTGACTGCAGACAAATCCTCCAGCACT
GCCTACATGCAGCTCAGCAGCCTGACATCTGATGACTCTGCGGTCTATTTCTGTGCAAG
AATCTACTATGGTAACTCCTTTGCTTACTGGGGCCAAGGGACTCTGGTCACTGTCTCTG
CA
**SEQ ID NO: 12 (VH + linker +VL CL6):** 
QVQLQQSGAELVRPGTSVKVSCKASGYAFTNYLIEWVKERPGQGLEWIGVINPGSGATNY
NEKFKGKATLTADKSSSTAYMQLSSLTSDDSAVYFCARIYYGNSFAYWGQGTLVTVSAGGG
GSGGGGSGGGGSGGGGSDIVMTQSPSSLTVTAGEKVTMNCKSSQSLLNSGSQKNYLTWY
QQKPGQPPKLLIFWASTRESGVPDRFTGSGSGTDFTLTISSVQAEDLAVYYCQNNYYYPLT
FGAGTKLELK
**SEQ ID NO: 44 (VL + linker +VH CL6):** 
DIVMTQSPSSLTVTAGEKVTMNCKSSQSLLNSGSQKNYLTWYQQKPGQPPKLLIFWASTRE
SGVPDRFTGSGSGTDFTLTISSVQAEDLAVYYCQNNYYYPLTFGAGTKLELKGGGGSGGG
GSGGGGSGGGGSQVQLQQSGAELVRPGTSVKVSCKASGYAFTNYLIEWVKERPGQGLE
WIGVINPGSGATNYNEKFKGKATLTADKSSSTAYMQLSSLTSDDSAVYFCARIYYGNSFAY
WGQGTLVTVSA
**SEQ ID NO: 13 (CAR CL6 VL+VH):** 
MALPVTGLLLSLGLLLHAARPTGDIVMTQSPSSLTVTAGEKVTMNCKSSQSLLNSGSQKNYL
TWYQQKPGQPPKLLIFWASTRESGVPDRFTGSGSGTDFTLTISSVQAEDLAVYYCQNNYYY
PLTFGAGTKLELKGGGGSGGGGSGGGGSGGGGSQVQLQQSGAELVRPGTSVKVSCKAS
GYAFTNYLIEWVKERPGQGLEWIGVINPGSGATNYNEKFKGKATLTADKSSSTAYMQLSSL
TSDDSAVYFCARIYYGNSFAYWGQGTLVTVSATTTPAPRPPTPAPTIASQPLSLRPEACRPA
AGGAVHTRGLDFACDIYIWAPLAGTCGVLLLSLVITLYCKRGRKKLLYIFKQPFMRPVQTTQE
EDGCSCRFPEEEEGGCELRVKFSRSADAPAYQQGQNQLYNELNLGRREEYDVLDKRRGR
DPEMGGKPQRRKNPQEGLYNELQKDKMAEAYSEIGMKGERRRGKGHDGLYQGLSTATKD
TYDALHMQALPPR
**SEQ ID NO: 45 (CAR CL6 VH + VL):** 
MALPVTGLLLSLGLLLHAARPTGQVQLQQSGAELVRPGTSVKVSCKASGYAFTNYLIEWVK
ERPGQGLEWIGVINPGSGATNYNEKFKGKATLTADKSSSTAYMQLSSLTSDDSAVYFCARIY
YGNSFAYWGQGTLVTVSAGGGGSGGGGSGGGGSGGGGSDIVMTQSPSSLTVTAGEKVT
MNCKSSQSLLNSGSQKNYLTWYQQKPGQPPKLLIFWASTRESGVPDRFTGSGSGTDFTLTI
SSVQAEDLAVYYCQNNYYYPLTFGAGTKLELKTTTPAPRPPTPAPTIASQPLSLRPEACRPA
AGGAVHTRGLDFACDIYIWAPLAGTCGVLLLSLVITLYCKRGRKKLLYIFKQPFMRPVQTTQE
EDGCSCRFPEEEEGGCELRVKFSRSADAPAYQQGQNQLYNELNLGRREEYDVLDKRRGR
DPEMGGKPQRRKNPQEGLYNELQKDKMAEAYSEIGMKGERRRGKGHDGLYQGLSTATKD
TYDALHMQALPPR
**CL3**
**SEQ ID NO: 14 (LCDR1):** QTIGTW
**SEQ ID NO: 15 (LCDR2):** AAT
**SEQ ID NO: 16 (LCDR3):** QQLYSSPHT
**SEQ ID NO: 17 (HCDR1):** GYSFTGYT
**SEQ ID NO: 18 (HCDR2):** INPYNGGT
**SEQ ID NO: 19 (HCDR3):** ARDQNYGSSYDALDY
**SEQ ID NO: 20 (VL amino acid sequence):** 
DIQMTQSPASQSASLGESVTITCLASQTIGTWLAWYQQKPGKSPQLLIYAATSLADGVPSRF
SGSGSGTKFSFKISSLQAEDFVSYYCQQLYSSPHTFGGGTKLEIK
**SEQ ID NO: 21 (VH amino acid sequence):** 
EVQLQQSGPELVKPGASMKISCKASGYSFTGYTMNWVKQSHGKNLEWIGLINPYNGGTTY
NQKFKGKATLTVDKSSSTAYMELLSLTSEDSAVYYCARDQNYGSSYDALDYWGQGTSVTV
SS
**SEQ ID NO: 22 (VL nucleotide sequence):** 
GACATTCAGATGACCCAGTCTCCTGCCTCCCAGTCTGCATCTCTGGGAGAAAGTGTCAC
CATCACATGCCTGGCAAGTCAGACCATTGGTACATGGTTAGCATGGTATCAGCAGAAAC
CAGGGAAATCTCCTCAGCTCCTGATTTATGCTGCAACCAGCTTGGCAGATGGGGTCCCA
TCAAGGTTCAGTGGTAGTGGATCTGGCACAAAATTTTCTTTCAAGATCAGCAGCCTACA
GGCTGAAGATTTTGTAAGTTATTACTGTCAACAACTTTACAGTAGTCCTCACACGTTCGG
AGGGGGGACCAAGCTGGAAATAAAA
**SEQ ID NO: 23 (VH nucleotide sequence):** 
GAGGTCCAGCTGCAACAGTCTGGACCTGAGCTGGTGAAGCCTGGAGCTTCAATGAAGA
TATCCTGCAAGGCTTCTGGTTACTCATTCACTGGCTACACCATGAACTGGGTGAAGCAG
AGCCATGGAAAGAACCTTGAGTGGATTGGACTTATTAATCCTTACAATGGTGGTACTACC
TACAACCAGAAGTTCAAGGGCAAGGCCACATTAACTGTAGACAAGTCATCCAGCACAGC
CTACATGGAGCTCCTCAGTCTGACATCTGAGGACTCTGCAGTCTATTACTGTGCAAGAG
ATCAGAACTACGGTAGTAGCTACGATGCTCTGGACTACTGGGGTCAAGGCACCTCAGTC
ACCGTCTCCTCA
**SEQ ID NO: 24 (VL + linker +VH CL3):** 
DIQMTQSPASQSASLGESVTITCLASQTIGTWLAWYQQKPGKSPQLLIYAATSLADGVPSRF
SGSGSGTKFSFKISSLQAEDFVSYYCQQLYSSPHTFGGGTKLEIKGGGGSGGGGSGGGGS
GGGGSEVQLQQSGPELVKPGASMKISCKASGYSFTGYTMNWVKQSHGKNLEWIGLINPYN
GGTTYNQKFKGKATLTVDKSSSTAYMELLSLTSEDSAVYYCARDQNYGSSYDALDYWGQG
TSVTVSS
**SEQ ID NO: 46 (VH + linker +VL CL3):** 
EVQLQQSGPELVKPGASMKISCKASGYSFTGYTMNWVKQSHGKNLEWIGLINPYNGGTTY
NQKFKGKATLTVDKSSSTAYMELLSLTSEDSAVYYCARDQNYGSSYDALDYWGQGTSVTV
SSGGGGSGGGGSGGGGSGGGGSDIQMTQSPASQSASLGESVTITCLASQTIGTWLAWYQ
QKPGKSPQLLIYAATSLADGVPSRFSGSGSGTKFSFKISSLQAEDFVSYYCQQLYSSPHTFG
GGTKLEIK
**SEQ ID NO: 25 (CAR CL3 VL+VH):** 
MALPVTGLLLSLGLLLHAARPTGDIQMTQSPASQSASLGESVTITCLASQTIGTWLAWYQQK
PGKSPQLLIYAATSLADGVPSRFSGSGSGTKFSFKISSLQAEDFVSYYCQQLYSSPHTFGGG
TKLEIKGGGGSGGGGSGGGGSGGGGSEVQLQQSGPELVKPGASMKISCKASGYSFTGYT
MNWVKQSHGKNLEWIGLINPYNGGTTYNQKFKGKATLTVDKSSSTAYMELLSLTSEDSAVY
YCARDQNYGSSYDALDYWGQGTSVTVSSTTTPAPRPPTPAPTIASQPLSLRPEACRPAAG
GAVHTRGLDFACDIYIWAPLAGTCGVLLLSLVITLYCKRGRKKLLYIFKQPFMRPVQTTQEED
GCSCRFPEEEEGGCELRVKFSRSADAPAYQQGQNQLYNELNLGRREEYDVLDKRRGRDP
EMGGKPQRRKNPQEGLYNELQKDKMAEAYSEIGMKGERRRGKGHDGLYQGLSTATKDTY
DALHMQALPPR
**SEQ ID NO: 47 (CAR CL3 VH+VL):** 
MALPVTGLLLSLGLLLHAARPTGEVQLQQSGPELVKPGASMKISCKASGYSFTGYTMNWVK
QSHGKNLEWIGLINPYNGGTTYNQKFKGKATLTVDKSSSTAYMELLSLTSEDSAVYYCARD
QNYGSSYDALDYWGQGTSVTVSSGGGGSGGGGSGGGGSGGGGSDIQMTQSPASQSAS
 
LGESVTITCLASQTIGTWLAWYQQKPGKSPQLLIYAATSLADGVPSRFSGSGSGTKFSFKIS
SLQAEDFVSYYCQQLYSSPHTFGGGTKLEIKTTTPAPRPPTPAPTIASQPLSLRPEACRPAA
GGAVHTRGLDFACDIYIWAPLAGTCGVLLLSLVITLYCKRGRKKLLYIFKQPFMRPVQTTQEE
DGCSCRFPEEEEGGCELRVKFSRSADAPAYQQGQNQLYNELNLGRREEYDVLDKRRGRD
PEMGGKPQRRKNPQEGLYNELQKDKMAEAYSEIGMKGERRRGKGHDGLYQGLSTATKDT
YDALHMQALPPR
**CL2**
**SEQ ID NO: 26 (LCDR1):** STISSNY
**SEQ ID NO: 27 (LCDR2):RTS**
**SEQ ID NO: 28 (LCDR3):** QQGDSIPLT
**SEQ ID NO: 29 (HCDR1):** GFTFSSYT
**SEQ ID NO: 30 (HCDR2):** ISSGGSYT
**SEQ ID NO: 31 (HCDR3):** TRIYYDSYYAMDY
**SEQ ID NO: 32 (VL amino acid sequence):** 
EIVLTQSPTTMAASPGEKITITCSASSTISSNYLHWYQQKPGFSPKLLIYRTSNLASGVPARFS
GSGSGTSYSLTIGTMEAEDVATYYCQQGDSIPLTFGAGTKLELK
**SEQ ID NO: 33 (VH amino acid sequence):** 
DVKLVESGGGLVKPGGSLKLSCAASGFTFSSYTMSWVRQTPEKRLEWVATISSGGSYTYY
PDSVKGRFTISRDNAKNTLYLQMSSLKSEDTAMYYCTRIYYDSYYAMDYWGQGTSVTVSS
**SEQ ID NO: 34 (VL nucleotide sequence):** 
GAAATTGTGCTCACCCAGTCTCCAACCACCATGGCTGCATCTCCCGGGGAGAAGATCA
CTATCACCTGCAGTGCCAGCTCAACTATAAGTTCCAATTACTTGCATTGGTATCAGCAGA
AGCCAGGATTCTCCCCTAAACTCTTGATTTATAGGACATCCAATCTGGCTTCTGGAGTCC
CAGCTCGCTTCAGTGGCAGTGGGTCTGGGACCTCTTACTCTCTCACAATTGGCACCATG
GAGGCTGAAGATGTTGCCACTTACTACTGCCAGCAGGGTGATAGTATACCGCTCACGTT
CGGTGCTGGGACCAAGCTGGAGCTGAAA
**SEQ ID NO: 35 (VH nucleotide sequence):** 
GACGTGAAGCTGGTGGAGTCTGGGGGAGGCTTAGTGAAGCCTGGAGGGTCCCTGAAA
CTCTCCTGTGCAGCCTCTGGATTCACTTTCAGTAGCTATACCATGTCTTGGGTTCGCCA
GACTCCGGAGAAGAGGCTGGAGTGGGTCGCAACCATTAGTAGTGGTGGTAGTTACACC
TACTATCCAGACAGTGTGAAGGGCCGATTCACCATCTCCAGAGACAATGCCAAGAACAC
CCTGTACCTGCAAATGAGCAGTCTGAAGTCTGAGGACACAGCCATGTATTACTGTACAA
 
GAATCTACTATGATTCCTACTATGCTATGGACTACTGGGGTCAAGGAACCTCAGTCACC
GTCTCCTCA
**SEQ ID NO: 36 (VL + linker +VH CL2):** 
EIVLTQSPTTMAASPGEKITITCSASSTISSNYLHWYQQKPGFSPKLLIYRTSNLASGVPARFS
GSGSGTSYSLTIGTMEAEDVATYYCQQGDSIPLTFGAGTKLELKGGGGSGGGGSGGGGSG
GGGSDVKLVESGGGLVKPGGSLKLSCAASGFTFSSYTMSWVRQTPEKRLEWVATISSGGS
YTYYPDSVKGRFTISRDNAKNTLYLQMSSLKSEDTAMYYCTRIYYDSYYAMDYWGQGTSVT
VSS
**SEQ ID NO: 48 (VH + linker +VL CL2):** 
DVKLVESGGGLVKPGGSLKLSCAASGFTFSSYTMSWVRQTPEKRLEWVATISSGGSYTYY
PDSVKGRFTISRDNAKNTLYLQMSSLKSEDTAMYYCTRIYYDSYYAMDYWGQGTSVTVSS
GGGGSGGGGSGGGGSGGGGSEIVLTQSPTTMAASPGEKITITCSASSTISSNYLHWYQQK
PGFSPKLLIYRTSNLASGVPARFSGSGSGTSYSLTIGTMEAEDVATYYCQQGDSIPLTFGAG
TKLELK
**SEQ ID NO: 37 (CAR CL2 VL+VH):** 
MALPVTGLLLSLGLLLHAARPTGEIVLTQSPTTMAASPGEKITITCSASSTISSNYLHWYQQK
PGFSPKLLIYRTSNLASGVPARFSGSGSGTSYSLTIGTMEAEDVATYYCQQGDSIPLTFGAG
TKLELKGGGGSGGGGSGGGGSGGGGSDVKLVESGGGLVKPGGSLKLSCAASGFTFSSYT
MSWVRQTPEKRLEWVATISSGGSYTYYPDSVKGRFTISRDNAKNTLYLQMSSLKSEDTAMY
YCTRIYYDSYYAMDYWGQGTSVTVSSTTTPAPRPPTPAPTIASQPLSLRPEACRPAAGGAV
HTRGLDFACDIYIWAPLAGTCGVLLLSLVITLYCKRGRKKLLYIFKQPFMRPVQTTQEEDGCS
CRFPEEEEGGCELRVKFSRSADAPAYQQGQNQLYNELNLGRREEYDVLDKRRGRDPEMG
GKPQRRKNPQEGLYNELQKDKMAEAYSEIGMKGERRRGKGHDGLYQGLSTATKDTYDAL
HMQALPPR
**SEQ ID NO: 49 (CAR CL2 VH+VL):** 
MALPVTGLLLSLGLLLHAARPTGDVKLVESGGGLVKPGGSLKLSCAASGFTFSSYTMSWVR
QTPEKRLEWVATISSGGSYTYYPDSVKGRFTISRDNAKNTLYLQMSSLKSEDTAMYYCTRIY
YDSYYAMDYWGQGTSVTVSSGGGGSGGGGSGGGGSGGGGSEIVLTQSPTTMAASPGEK
ITITCSASSTISSNYLHWYQQKPGFSPKLLIYRTSNLASGVPARFSGSGSGTSYSLTIGTMEA
EDVATYYCQQGDSIPLTFGAGTKLELKTTTPAPRPPTPAPTIASQPLSLRPEACRPAAGGAV
HTRGLDFACDIYIWAPLAGTCGVLLLSLVITLYCKRGRKKLLYIFKQPFMRPVQTTQEEDGCS
CRFPEEEEGGCELRVKFSRSADAPAYQQGQNQLYNELNLGRREEYDVLDKRRGRDPEMG
 
GKPQRRKNPQEGLYNELQKDKMAEAYSEIGMKGERRRGKGHDGLYQGLSTATKDTYDAL
HMQALPPR
**General sequences (CAR Backbone)**
   **SEQ ID NO: 38 (CD8a signal peptide):**
   MALPVTGLLLSLGLLLHAARPTG
**SEQ ID NO: 39 (linker)**
   GGGGSGGGGSGGGGSGGGGS
**SEQ ID NO: 40 (CD8 hinge)**
   TTTPAPRPPTPAPTIASQPLSLRPEACRPAAGGAVHTRGLDFACD
**SEQ ID NO: 41 (CD8 Transmembrane)**
   IYIWAPLAGTCGVLLLSLVITLYC
**SEQ ID NO: 42 (4-1BB)**
   KRGRKKLLYIFKQPFMRPVQTTQEEDGCSCRFPEEEEGGCEL
**SEQ ID NO: 43 (CD3z)**

RVKFSRSADAPAYQQGQNQLYNELNLGRREEYDVLDKRRGRDPEMGGKPQRRKNPQEGL
YNELQKDKMAEAYSEIGMKGERRRGKGHDGLYQGLSTATKDTYDALHMQALPPR

### EXAMPLES

### Material and Methods

### Mouse immunization

Five-week-old Mus musculus mice from the BALB/c strain were used to obtain anti-human Claudin 18.2 monoclonal antibodies. Mice were housed in the animal facility at the University of Vigo under standard feeding, water, and light-dark cycle conditions. The mice were immunized with human Claudin 18.2 full length protein in Virus-Like Particle (VLP) format (#CL2-H52P7, ACROBiosystems). Mice received three intraperitoneal immunizations using a 1 mL syringe with a 20G needle. The first immunization was performed with Complete Freund's Adjuvant (CFA) (#F5881, Sigma). 50 µg of antigen were resuspended in Phosphate-Buffered Saline (PBS) and slowly mixed with an equal volume of the corresponding adjuvant to achieve a homogeneous mixture. This process was carried out under sterile conditions in a laminar flow cabinet. After 21 days, the mice received a second immunization with 25 µg of antigen, using the same procedure as the first immunization, with the exception that CFA was replaced by Incomplete Freund's Adjuvant (IFA) (#F5506, Sigma). To assess the humoral immune response, sera samples were collected from the immunized mice 14 days after the second immunization and analysed by ELISA. Blood samples were obtained by submandibular puncture. The collected bloods (200-500 µL) were transferred to Eppendorf tubes and incubated at room temperature for one hour to allow clot formation. Samples were centrifuged at 1,500 x g for 15 minutes at 4°C in a microcentrifuge, and the serum from each mouse was collected while discarding the precipitate. Those mice showing strong humoral responses, received a final booster immunization with 10 µg of antigen without adjuvant, administered intraperitoneally five days before sacrifice.

### Hybridoma generation

### Culture Media

- **Complete medium:** RPMI 1640 (#15-040-CV, Corning), supplemented with 100 U/mL penicillin and 100 µg/mL streptomycin (#15140122, Gibco), with 10-20% fetal bovine serum (FBS) (#A5256801, Gibco).
- **HAT medium:** Complete medium supplemented with 1X HAT (Hypoxanthine, Aminopterin, Thymidine) (#H0262, Sigma).
- **HT medium:** Complete medium supplemented with 1X HT (Hypoxanthine, Thymidine) (#H0137, Sigma).

### Spleen extraction

Five days after the final immunization, the chosen mouse was euthanized by an intraperitoneal anaesthetic overdose of ketamine (75 mg/kg) and medetomidine (1 mg/kg). The mouse was placed in a beaker containing 70% ethanol, and the spleen was removed using forceps and scissors, then placed in a Petri dish containing complete medium. To obtain a cell suspension, medium was passed through the spleen using a 1 mL syringe fitted with a 20G needle. The spleen was then placed on a sterile cell strainer and dissociated with the plunger. The cell suspension was transferred to a 15 mL tube and allowed to rest on ice for two minutes allowing connective tissue fragments to settle. Spleen cells were transferred to another 15 mL tube and centrifuged at 1,200 rpm at 4°C for 5 minutes. The supernatant was discarded, and the cells were resuspended in 10 mL of complete medium and kept on ice for counting with trypan blue.

### Myeloma cells

The SP2 myeloma cell line (Kohler and Milstein, 1976) was used for the fusion. This line, derived from plasmacytoma cells, does not produce antibodies and lacks the enzyme hypoxanthine-guanine phosphoribosyltransferase (HGPRT-), preventing its growth in hypoxanthine-aminopterin-thymidine medium (HAT). To ensure enough viable myeloma cells, the culture was initiated approximately ten days prior to fusion. Cells were cultured in complete medium under sterile conditions in a vertical laminar flow cabinet, incubated at 37°C in a humidified atmosphere with 5% CO₂. They were maintained in sterile plastic culture flasks of 25 or 75 cm² with a membrane-vented cap. The culture medium was renewed regularly, and cell growth and absence of contamination were checked daily using a light microscope.

### Hybridomas

For hybridoma production, SP2 myeloma cells were fused with mouse spleen cells. This procedure was performed under sterile conditions in a vertical laminar flow cabinet. Spleen and myeloma cells were combined in a 50 mL tube at a ratio of 1 myeloma cell to 10 spleen cells in FBS-free medium and centrifuged at 1,200 rpm for 5 minutes at room temperature. All subsequent steps were carried out at 37°C in a water bath. The pellet was resuspended, and 1 mL of 40% w/v polyethylene glycol 1500 (PEG 1500) (#10783641001, Roche) in PBS, prewarmed to 37°C, was added dropwise while gently mixing with the glass pipette. Culture medium at 37°C was gradually added to reach a final volume of 15 mL. The mixture was centrifuged at 1,200 rpm for 5 minutes at room temperature, and the pellet was resuspended in 200 mL of selective HAT medium supplemented with 20% FBS. This cell suspension was distributed into eight 24-well plates. After 14 days of culture, when cell colonies were visible, supernatants were screened by ELISA to detect specific antibodies. Colonies present in the positive wells were individually transferred to 96-well plates using a binocular microscope and a micropipette, with 200 µL of selective HAT medium added to each well. The medium was replaced every 2-3 days to eliminate any residual antibodies, ensuring that detected antibodies were secreted by the hybridoma cells of each colony. Several days after the last medium change, supernatants were screened by ELISA for specific antibodies. Hybridomas secreting specific immunoglobulins were expanded in 24-well plates. Selective medium was gradually replaced with HT medium containing 20% FBS and then with complete medium containing 20% FBS, with FBS concentration gradually reduced to 10%. At each step, half of the cells from each well were frozen.

### Detection of Specific Antibodies

### ELISA for Claudin 18.2 protein

The presence of Claudin 18.2-specific antibodies in mouse sera and hybridoma supernatants was analysed using an indirect ELISA. Flat-bottom 96-well plates (#439454, Thermo Scientific) were coated with 50 µL per well of Recombinant Human Claudin 18.2-VLP (#CSB-MP005498HU(A5), Cusabio) or VLP (isotype control from the same supplier), diluted in carbonate-bicarbonate buffer pH 9.6 at a concentration of 5 µg/mL and incubated overnight at 4°C. The next day, the wells were blocked with 125 µL of PBS-BSA (2%) for 2 hours at room temperature to prevent non-specific binding. The plates were then incubated for 2 hours at room temperature with 50 µL of diluted serum or undiluted hybridoma supernatant. The serum dilutions used were 1:2,000, 1:4,000 and 1:8,000 in PBS-BSA (1%). After three washes with PBS-Tween 20 (0.1%), 50 µL of rabbit anti-mouse IgG (H+L) polyclonal secondary antibodies conjugated to biotin (#PA1-28567, Invitrogen) diluted 1:1,000 in PBS-BSA (1%) were added. Following 1 hour of incubation at room temperature, the wells were washed three times with PBS-Tween 20 (0.1%), and 50 µL of Horse radish peroxidase (HRP)-conjugated streptavidin (#405210, BioLegend) diluted 1:1,000 in PBS-BSA (1%) were added. Since one biotin molecule can bind up to four streptavidin molecules, this method allows significant signal amplification. After 30 minutes of incubation at room temperature, the wells were washed three times with PBS-Tween 20 (0.1%). For colorimetric detection, 50 µL of 3,3',5,5'-Tetramethylbenzidine (TMB) substrate (#555214, BD Biosciences) were added by mixing equal parts of Reagent A and Reagent B. The colour reaction was stopped after 10 minutes by adding 25 µL of 1M sulfuric acid, and the optical density was measured at 450 nm using a microplate reader (EnVision). Wells containing only PBS were used as blanks. A mouse anti-CLDN18.2 recombinant antibody (#HPAB-0120-YJ, Creative Biolabs) was used as positive control.

### Flow Cytometry

The ability of the antibodies produced by different hybridomas to recognize tumoral cells expressing Claudin 18.2 was assessed using flow cytometry. The cells used in this assay were MIA PaCa-2, a human pancreatic cancer cell line. This line was transfected with the Claudin 18 (CLDN18) (NM_001002026) Human Tagged ORF Clone plasmid (#RC219760, OriGene) to increase the expression of this protein.

To transfect MIA PaCa-2 cells with the Claudin 18.2 plasmid, cells were seeded 24 hours before transfection at a density of 200,000 cells per well in a 6-well plate containing 3 mL of Dulbecco's Modified Eagle Medium (DMEM) (#11965092, Gibco) supplemented with 100 U/mL penicillin and 100 µg/mL streptomycin (#15140122, Gibco), along with 10% fetal bovine serum (FBS) (#A5256801, Gibco). After this period, 1 µg of plasmid DNA was diluted in 250 µL of Opti-MEM (#11058021, Gibco) without serum or antibiotics and gently mixed using a vortex. Subsequently, 3 µL of Turbofectin 8.0 (#TF81001, Origene) were added to the diluted DNA and gently pipetted to ensure thorough mixing. The mixture was then incubated for 15 minutes at room temperature. For transfection, the cell culture medium was replaced with Opti-MEM without serum or antibiotics and the prepared complex was added dropwise to the cells, followed by gentle forward-backward and left-right movements of the plate to ensure uniform distribution. The cells were incubated at 37°C with 5% CO₂ for 6 hours, after which the medium was replaced with selection medium. The selection medium was Dulbecco's Modified Eagle Medium (DMEM) (#11965092, Gibco) supplemented with 100 U/mL penicillin and 100 µg/mL streptomycin (#15140122, Gibco), 10% fetal bovine serum (FBS) (#A5256801, Gibco) and 600 µg/mL G418 (Geneticin) (#ant-gn-1, InvivoGen). Cells were maintained under selection for at least one week, with medium changes every 2-3 days. Once selection was complete, cells were frozen and later analysed by flow cytometry.

Flow cytometry was used to evaluate the membrane expression of Claudin 18.2 in transfected cells. For each sample, 1,000,000 cells were resuspended in 1 mL of PBS and centrifuged at 1,200 rpm for 5 minutes at 4°C. The supernatant was discarded, and the pellet was washed with 1 mL of PBS, followed by a second centrifugation under the same conditions. The pellet was resuspended in 100 µL of Zombie Yellow Viability dye (#423103, BioLegend) at a dilution of 1:200 in PBS and incubated for 15 minutes at room temperature in the dark. The cells were then centrifuged at 1,200 rpm for 5 minutes at 4°C. The supernatant was discarded, and a second wash was performed with 1 mL of PBS, followed by centrifugation. Subsequently, 100 µL of PE-labeled anti-Claudin-18 isoform 2 antibody (#101676, BPS Bioscience) diluted 1:20 in PBS-BSA (0.5%) were added to the cells, which were then incubated for 30 minutes at room temperature in the dark. Following another centrifugation and a final wash with 1 mL of PBS-BSA (0.5%) under the same centrifugation conditions, the cells were resuspended in 100 µL of PBS-BSA (0.5%) for acquisition on a CytoFLEX S cytometer (Beckman Coulter). Cells incubated with PBS-BSA (0.5%) instead of the primary antibody were used as a negative control. Untransfected MIA PaCa-2 cells incubated with the PE-labeled anti-Claudin 18.2 antibody were also used as a negative control.

After establishing a cell line with high Claudin 18.2 expression, hybridoma supernatants were tested on it. For each well of a 96-well round-bottom plate, 250,000 cells resuspended in 200 µL of PBS were added. The plate was centrifuged at 1,200 rpm for 5 minutes at 4°C. The supernatant was discarded, and 200 µL of PBS was added to wash the cells, followed by a second centrifugation under the same conditions. The supernatant was then removed, 50 µL of Zombie Yellow Viability dye (#423103, BioLegend) diluted 1:200 in PBS were added, and the cells were incubated for 15 minutes at room temperature in the dark.After centrifuging the cells at 1,200 rpm for 5 minutes at 4°C and discarding the supernatant, a second wash was performed with 200 µL of PBS, again by centrifugation. Subsequently, 50 µL of the hybridoma supernatants were added to the cells, which were then incubated for 30 minutes at room temperature in the dark. After an additional centrifugation and removal of the supernatant, the cells were washed with 200 µL of PBS-BSA (0.5%) under the same conditions.

After removing the supernatant, 50 µL of rabbit anti-mouse IgG (H+L) biotin-conjugated secondary antibodies (#PA1-28567, Invitrogen), diluted 1:1,500 in PBS-BSA (0.5%) were added and incubated for 30 minutes at 4°C in the dark. The cells were washed again with 200 µL of PBS-BSA (0.5%) using the same centrifugation conditions. After discarding the supernatant, 50 µL of FITC-conjugated streptavidin (#405201, BioLegend) diluted 1:200 in PBS-BSA (0.5%) were added and incubated for 30 minutes at 4°C in the dark. Following another centrifugation and a final wash with 200 µL of PBS-BSA (0.5%) under the same centrifugation conditions, the cells were resuspended in 50 µL of PBS-BSA (0.5%). Cell analysis was performed using a CytoFLEX S cytometer (Beckman Coulter). Cells incubated with RPMI and an unspecific IgG, mouse IgG1-UNLB (15H6) (#0102-01, Southern Biotech) as primary antibodies were used as negative controls.

### Cloning by limiting dilution

To ensure monoclonality, stable antibody-secreting hybridomas specific for Claudin 18.2 and capable of recognizing tumor cells were cloned by limiting dilution in 96-well plates. Starting with 1,000 cells in the first column, serial 1:2 dilutions were performed across the remaining columns, with cells in a final volume of 200 µL per well. The expected cell number in the final column (column 12) was 0 or 1 cell per well. Plates were incubated at 37°C with 5% CO₂. Supernatants from clones were re-tested by ELISA or flow cytometry to confirm continued recognition of the target protein.

### Analysis of antibody isotype

Following confirmation of antibody production and monoclonality, the isotype of each antibody was determined using the Pierce^{™} Rapid Antibody Isotyping Kit - Mouse (#26178, Thermo Fisher Scientific).

### Antibody purification from hybridoma supernatants

To purify the antibodies produced by the hybridomas of interest, the cells were cultured and progressively amplified, with the concentration of FBS gradually reduced from 10% to 5%, and finally to 0%. Once the hybridomas had grown sufficiently and were adapted to the FBS-free medium, antibody production was allowed until complete cell death occurred, achieving maximum antibody production.

The supernatant from each hybridoma was diluted in a 1:1 ratio with binding buffer (20 mM sodium phosphate, pH 7.0) and filtered through a 0.22 µm filter to remove any residual particles or contaminants. For IgG purification, HiTrap Protein G HP columns (#17040401, Cytiva) were used. The syringe and pump tubing were filled with binding buffer (pH 7.0), and the pump flow rate was set to 1 mL/min. The pump was then connected to the Protein G column, and the entire diluted supernatant was passed through it. The column was subsequently washed with binding buffer. To elute the antibodies, 0.1 M Glycine-HCl (pH 2.7) was passed through the column, and the eluted antibodies were collected in separate fractions. Each fraction contained 50-100 µL of 1 M Tris-HCl (pH 9.0) to neutralize the pH and ensure optimal preservation of the purified antibodies.

### Peptide binding assessment

The binding of purified monoclonal antibodies to a peptide derived from human Claudin 18.2 was analysed using an indirect ELISA. The peptide was designed based on one of the two extracellular regions of human Claudin 18.2, which differs from Claudin 18.1. Its amino acid sequence is **QWSTQDLYNNPVTAVFNYQ.** It was synthesized and conjugated to Keyhole limpet haemocyanin (KLH) as carrier by Proteogenix, with a terminal cysteine added to enable conjugation. Flat-bottom 96-well plates (#055206, Nunc) were coated with 50 µL per well of Claudin 18.2-peptide conjugated to KLH, diluted in PBS buffer at a concentration of 100 µg/mL and incubated overnight at 4°C. The next day, the wells were blocked with 125 µL of PBS-skim milk (5%) for 2 hours at room temperature to prevent non-specific binding. The plates were then incubated for 2 hours at room temperature with 50 µL of purified mAb diluted in PBS-skim milk (2%). After three washes with PBS-Tween 20 (0.1%), 50 µL of goat anti-mouse IgG polyclonal secondary antibodies conjugated to horse rabbish peroxidase (HRP) (#1030-05, SouthernBiotech) diluted 1:2,000 in PBS-skim milk (2%) were added. Following 1 hour of incubation at room temperature, the wells were washed three times with PBS-Tween 20 (0.1%), and 50 µL of 3,3',5,5'-Tetramethylbenzidine (TMB) substrate (#555214, BD Biosciences) were added by mixing equal parts of Reagent A and Reagent B for colorimetric detection. The colour reaction was stopped after 10 minutes by adding 25 µL of 1M sulfuric acid, and the optical density was measured at 450 nm using a microplate reader (EnVision). Wells containing only PBS and an unspecific IgG, mouse IgG1-UNLB (15H6) (#0102-01, SouthernBiotech) were used as negative controls.

### Results

### Specific Immune response anti-Claudin 18.2

To evaluate the immunogenic response to Claudin 18.2-VLP, serum samples collected after mouse immunization were analysed using indirect ELISA. After two initial immunizations, the analysis revealed that the immunized mouse generated specific antibodies against Claudin 18.2-VLP, as illustrated in **Figure 1****.** The results compare the absorbance values between immunized and non-immunized mice.

### Monoclonal antibodies

Hybridomas were generated by fusing spleen cells from the immunized mouse with myeloma cells. Fourteen days post-fusion, several hybridomas were isolated and screened to identify those producing antibodies specific to Claudin 18.2. Subsequent ELISA screenings revealed that three hybridomas, named as **CL2, CL3 and CL6,** produced antibodies that recognized Claudin 18.2-VLP but not the VLP, confirming their specificity for the target protein. The absorbance values for these interactions, obtained from some hybridomas, are shown in **Figure 2****.**

Flow Cytometry analysis further revealed that these three hybridomas produced antibodies capable of binding Claudin 18.2 expressed on the membrane of transfected MIA PaCa-2 cells, indicating specific recognition of the extracellular region of the protein (**Figure 3**).

### Characterization of selected monoclonal antibodies

After confirming monoclonality, the immunoglobulin isotype of the three selected antibodies was determined. Isotyping results revealed that CL2 and CL6 were **IgG1**, while CL3 was **IgG2a.**

### Peptide binding assessment

After purifying the monoclonal antibodies by affinity chromatography, their ability to bind to a specific extracellular region of human Claudin 18.2 was analysed by indirect ELISA against the peptide conjugated to KLH. This ELISA screening demonstrated that all three selected mAbs were capable of recognizing the peptide. The absorbance values for these interactions are shown in **Figure 4****.**

### Bibliography

Köhler G, Milstein C. Derivation of specific antibody-producing tissue culture and tumor lines by cell fusion. Eur J Immunol. 1976 Jul;6(7):511-9. doi: 10.1002/eji.1830060713

## Claims

1. **A Claudin 18.2 targeting moiety** that specifically binds to the amino acid sequence of SEQ ID NO: 1.

2. A Claudin 18.2 targeting moiety according to claim 1, comprising an antibody, F(ab')2, Fab, scFab or scFv, comprising an VL and VH domain, wherein:
- the VL domain comprises a sequence with at least 85% sequence identity to SEQ ID NO: 8 (**CL6 VL**), and wherein said VL domain comprises CDR regions consisting of LCDR1 as set forth in SEQ ID NO: 2, LCDR2 as set forth in SEQ ID NO: 3, LCDR3 as set forth in SEQ ID NO: 4; and wherein the VH domain comprises a sequence with at least 85% sequence identity to SEQ ID NO: 9 (**CL6 VH**), and wherein said VH domain comprises CDR regions consisting of HCDR1 as set forth in SEQ ID NO: 5, HCDR2 as set forth in SEQ ID NO: 6, and HCDR3 as set forth in SEQ ID NO: 7, or
- the VL domain comprises a sequence with at least 85% sequence identity to SEQ ID NO: 20 (**CL3 VL)**, and wherein said VL domain comprises CDR regions consisting of LCDR1 as set forth in SEQ ID NO: 14, LCDR2 as set forth in SEQ ID NO: 15, LCDR3 as set forth in SEQ ID NO: 16; and wherein the VH domain comprises a sequence with at least 85% sequence identity to SEQ ID NO: 21 (**CL3 VH**), and wherein said VH domain comprises CDR regions consisting of HCDR1 as set forth in SEQ ID NO: 17, HCDR2 as set forth in SEQ ID NO: 18, and HCDR3 as set forth in SEQ ID NO: 19, or
- the VL domain comprises a sequence with at least 85% sequence identity to SEQ ID NO: 32 (**CL2 VL**), and wherein said VL domain comprises CDR regions consisting of LCDR1 as set forth in SEQ ID NO: 26, LCDR2 as set forth in SEQ ID NO: 27, LCDR3 as set forth in SEQ ID NO: 28; and wherein the VH domain comprises a sequence with at least 85% sequence identity to SEQ ID NO: 33 (**CL2 VH**), and wherein said VH domain comprises CDR regions consisting of HCDR1 as set forth in SEQ ID NO: 29, HCDR2 as set forth in SEQ ID NO: 30, and HCDR3 as set forth in SEQ ID NO: 31.

3. The Claudin 18.2 targeting moiety of claims 1 or 2, comprising
a. a VL domain consisting of SEQ ID NO: 8 and a VH domain consisting of SEQ ID NO: 9;
b. a VL domain consisting of SEQ ID NO: 20 and a VH domain consisting of SEQ ID NO: 21; or
c. a VL domain consisting of SEQ ID NO: 32 and a VH domain consisting of SEQ ID NO: 33.

4. The Claudin 18.2 targeting moiety according to any one of the preceding claims, wherein the Claudin targeting moiety comprises the amino acid sequence of SEQ ID NO: 12, SEQ ID NO: 24, SEQ ID NO: 36, SEQ ID NO: 44, SEQ ID NO: 46, SEQ ID NO: 48.

5. **A chimeric antigen receptor** (CAR) comprising:
a. an extracellular domain comprising a Claudin 18.2 targeting moiety according to any one of claims 1 to 4;
b. a transmembrane domain; and
c. an intracellular signaling domain.

6. The Claudin 18.2 according to claim 5, wherein
a. the transmembrane domain comprises the transmembrane domain of CD28, CD3, CD45, CD4, CD8, CD9, CD16, CD22, CD33, CD37, CD64, CD80, CD86, CD134, CD137, or CD154, preferably wherein the transmembrane domain comprises the transmembrane domain of CD8; and/or
b. the intracellular signaling domain comprises the intracellular domain of CD3ζ, FcRγ, CD3γ, CD3δ, CD3ε, CD5, CD22, CD79a, CD79b or CD66b, preferably of CD3ζ; and/or
c. the CAR further comprises a costimulatory signaling domain, preferably the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276, preferably of CD137.

7. The CAR according to any one of claims 5 or 6 consisting of the amino acid sequence according to SEQ ID NO: 13, SEQ ID NO: 25, SEQ ID NO: 37, SEQ ID NO: 45, SEQ ID NO: 47 or SEQ ID NO: 49.

8. **A nucleic acid** encoding the Claudin18.2 targeting moiety as defined in any one of claims 1 to 4, or encoding the CAR as defined in any one of claims 5 to 7.

9. **A cell** comprising the Claudin 18.2 targeting moiety as defined in any one of claims 1 to 4, the CAR as defined in any one of claims 5 to 7, or the nucleic acid as defined in claim 8.

10. A **pharmaceutical composition** comprising the Claudin 18.2 targeting moiety as defined in any one of claims 1 to 4, the CAR as defined in any one of claims 5 to 7, the nucleic acid as defined in claim 8, or a plurality of cells as defined in claim 9, and a pharmaceutically acceptable carrier or diluent.

11. The Claudin 18.2 targeting moiety as defined in any one of claims 1 to 4, the CAR as defined in any one of claims 5 to 7, the nucleic acid as defined in claim 8, the cell as defined in claim 9, or the pharmaceutical composition as defined in claim 10, **for use** in medicine or as a medicament.

12. The Claudin 18.2 targeting moiety as defined in any one of claims 1 to 4, the CAR as defined in any one of claims 5 to 7, the nucleic acid as defined in claim 8, the cell as defined in claim 9, or the pharmaceutical composition as defined in claim 10, **for** use in a method of diagnosing, treating or preventing a Claudin 18.2-positive cancer, wherein the method comprises administering the cell or composition to a patient in need thereof.

13. The Claudin 18.2 targeting moiety, the CAR, the nucleic acid, the cell, or the pharmaceutical composition for use as defined in claim 12, wherein the Claudin 18.2-positive cancer is gastric cancer or pancreas cancer or esophageal cancer.

14. The Claudin 18.2 targeting moiety as defined in any one of claims 1 to 4, the CAR as defined in any one of claims 5 to 7, the nucleic acid as defined in claim 8, the cell as defined in claim 9, or the pharmaceutical composition as defined in claim 10, **for use** in the diagnostic field.
